(19) Europäisches Patentamt
European Patent Office
Office européen des brevets

(11) EP 2 088 194 B1

(12) # EUROPEAN PATENT SPECIFICATION

(45) Date of publication and mention of the grant of the patent:
**12.01.2011 Bulletin 2011/02**

(21) Application number: **07830755.0**

(22) Date of filing: **29.10.2007**

(51) Int Cl.:
***C12N 15/09** (2006.01)* ***C12Q 1/68** (2006.01)*

(86) International application number:
**PCT/JP2007/071021**

(87) International publication number:
**WO 2008/053834 (08.05.2008 Gazette 2008/19)**

(54) **METHOD FOR SELECTING HOP LINE AND BREEDING MARKER AND PRIMER SET USED FOR SELECTING HOP LINE**

VERFAHREN ZUR AUSWAHL EINER HOPFENLINIE SOWIE ZUR AUSWAHL DER HOPFENLINIE VERWENDETER ZÜCHTUNGSMARKER UND PRIMERSATZ

PROCÉDÉ DE SÉLECTION D'UNE LIGNÉE DE HOUBLON ET ENSEMBLE D'AMORCES ET MARQUEUR DE SÉLECTION UTILISÉ POUR SÉLECTIONNER LA LIGNÉE DE HOUBLON

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.10.2006 JP 2006294798**

(43) Date of publication of application:
**12.08.2009 Bulletin 2009/33**

(73) Proprietor: **Sapporo Breweries Limited**
**Tokyo 150-8522 (JP)**

(72) Inventors:
- **OKADA, Yukio**
**Shibuya-ku**
**Tokyo 150-8522 (JP)**
- **KOIE, Koichiro**
**Shibuya-ku**
**Tokyo 150-8522 (JP)**

(74) Representative: **Vossius & Partner**
**Siebertstrasse 4**
**81675 München (DE)**

(56) References cited:
**WO-A1-02/088350 JP-A- 2006 075 030**

- **CERENAK ANDREJA; SATOVIC ZLATKO; JAVORNIK BRANKA: "Genetic mapping of hop (Humulus lupulus L.) applied to the detection of QTLs for alpha-acid content" GENOME, vol. 49, no. 5, May 2006 (2006-05), pages 485-494, XP002547531 ISSN: 0831-2796**
- **KOIE K; INABA A; OKADA Y; KANEKO T; ITO K: "Construction of the genetic linkage map and QTL analysis on hop (Humulus lupulus L)" ACTA HORTICULTURAE, WAGENINGEN, NL, vol. 668, 1 January 2005 (2005-01-01), pages 59-66, XP008112287 ISSN: 0567-7572**
- **MURAKAMI A: "Genetic Distance Based on RAPD and its Application to Hop Breeding" BREEDING SCIENCE, JAPANESE SOCIETY OF BREEDING, TOKYO, JP, vol. 50, 1 January 2000 (2000-01-01), pages 23-28, XP003024705 ISSN: 1344-7610**
- **MURAKAMI A.: 'Genetic Distance Based on RAPD and its Application to Hop Breeding' BREEDING SCIENCE vol. 50, 2000, pages 23 - 28, XP003024705**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Sapporo Breweries Ltd.
<120> Method for selecting hop line and breeding marker and primer set used for selecting hop line
<130> R1783 EP s3
<140> EP 07 83 0755.0
<141> 2007-10-29
<150> PCT/JP2007/071021
<151> 2007-10-29
<150> JP 2006-294798
<151> 2006-10-30
<160> 8
<170> PatentIn version 3.1
<210> 1
<211> 1684
<212> DNA

<213> Humulus lupulus
<400> 1

actagtttct ctccacaatc tctgctattg tgcgcactcc ttcctactcc catgtcgatc 60

actttccacc tctgccggat cccaacccgt accgacctcg ccttgccgga ggccctctcc 120

agaaccggac ccttccgctg ccgaaaacct ttcgttgtcc gatgctccgg cgagtcatct 180

tccacggcag gagattccga cttcgatgcg aaagtgttcc gtaagaactt ggttcgaagc 240

aagaactaca atcggaaagg ttttggccat aaggaagaga ctcttcagct catgaacagc 300

gagtacacca gtgatattat aaagactttg aaggataacg ggaacgagta tagctggggg 360

aacgtgactg tgaaattggc cgaagcttat gggttttgct ggggtgtgga gcgagctgtc 420

cagattgctt acgaagcaag aaaacagttc cccgaagaaa aaatttggat tacaaacgaa 480

atcattcaca atcccacagt caacaagagg ctagaggaaa tgaaagtaga aaacattccg 540

attggtgaag ggaggaaaca attcgagatt gtaaacgagg gtgatgttgt gatattgcct 600

gcgtttggtg ctggagtgga tgagatgttg gctttgagtg ataggaatgt tcaaattgtt 660

gataccacat gtccatgggt ttccaaggtt tggaatacag tcgagaaaca taagaaggcc 720

gaatacactt ccattattca tggtaaatat gctcatgagg agactattgc aactgcatct 780

tttgctggaa cttacattat tgtaaagaac atgaaagagg caatgtatgt gtgtgattac 840

attctcggcg gtcaactgga tggatctagc tcaactagag aggagtttat ggagaaattt 900

aagaatgctg tttctaaggg attcgatcct gacgaacatc ttgtgaaggt tggtattgcg 960

aatcagacca caatgctcaa gggggaaacc gaagagattg ggaaactggt tgagagggct 1020

atgatgcaaa agtatggagt ggaaaacatt aatgaacact tccaaagctt taacacaatt 1080

tgcgatgcga cccaagagcg tcaagatgca atgtacaagt tggtggagga acctattgat 1140

cttatgttag ttgttggagg atggaactcg agtaacacct ctcatctaca agagattgca 1200

gaggaacgtg gtattccctc gtattggatc gacagtgaac agagaatagg tcctggaaac 1260

aaaatagcct acaagctaaa tcatggagag ttggttgaga aagagaactg gttaccagag 1320

ggtcccatca cgattggtgt aacatcaggt gcttctactc cagataaggt tgttgaagat 1380

gttctcatca aggtgtttga ccttaaacgc gaagaagctt tgcaacttgc ttagtttaag 1440

ttagactcaa tgtttgtaca tcaccagaca gcgctatcat atagcattat tattgtattt 1500

ctaggatgaa atgttgggtc cttgttatgc aagagctcca tgtatctaag aatattaatc 1560

gtcgtatagt agtagaaatc acgttgttat tattgaaagg gatctaataa gttctttgaa 1620

tctagatagc agccatagcc tgcaataatg cgtttaattg ctgacacaat aatgatattc 1680

cacc 1684

<210> 2
<211> 25
<212> DNA
<213> Artificial
<220>
<221> source
<223> /note="Description of artificial sequence: primer"
<400> 2
actagtttct ctccacaatc tctgc 25
<210> 3
<211> 25
<212> DNA
<213> Artificial
<220>
<221> source
<223> /note="Description of artificial sequence: primer"
<400> 3
ggtggaatat cattattgtg tcagc 25
<210> 4
<211> 3460
<212> DNA
<213> Humulus lupulus
<400> 4

```
actagtttct ctccacaatc tctgctattg tgcgcactcc ttcctactcc catgtcgatc     60
actttccacc tctgccggat cccaacccgt accgacctcg ccttgccgga ggccctctcc    120
agaaccggac ccttccgctg ccgaaaacct ttcgttgtcc gatgctccgg cgagtcatct    180
tccacggcag gagattccga cttcgatgcg aaagtgttcc gtaagaactt ggttcgaagc    240
```

aagaactaca atcggaaagg ttttggccat aaggaagaga ctcttcagct catgaacagc 300
gagtacacca gtatgctgtg cttcgattcc tcaatttgtt tataatgaat cgtaattatt 360
tagtttgatt aaataattaa tttactggaa aacaaaattc aggtgatatt ataaagactt 420
tgaaggataa cgggaacgag tatagctggg ggaacgtgac tgtgaaattg gccgaagctt 480
atgggttttg ctggggtgtg gagcgagctg tccagattgc ttacgaagca agaaaacagt 540
tccccgaaga aaaaatttgg attacaaacg aaatcattca caatcccaca gtcaacaagg 600
tttaatcttc attttatttt ttgattaatg ttagtgtttt cttcttcgta atggctcaat 660
ttatatattt attaatatta ttatgattat ttggatagag gctagaggaa atgaaagtag 720
aaaacattcc gattggtgaa gggaggaaac aattcgagat tgtaaacgag ggtgatgttg 780
tgatattgcc tgcgtttggt gctggagtgg atgagatgtt ggctttgagt gataggaatg 840
ttcaaattgt tgataccaca tgtccatggg tttccaaggt aaccttttc tatttttatt 900
tttttaataa aagattttta aaatatggga tattgtttaa attatatgtt attaatggtt 960
ttggttgcaa atgctctggt ttgaaggttt ggaatacagt cgagaaacat aagaaggccg 1020
aatacacttc cattattcat ggtaaatatg ctcatgagga gactattgca actgcatctt 1080
ttgctggaac ttacattatt gtaaagaaca tgaaagaggt aacaactttt tctcttatga 1140
caaacaagtt ttttggttta ttctttgtgc tgtagatttt gatggaatct ttattgtctt 1200
aaattcaggc agtgtatgtg tgtgattaca ttctcggcgg tcaactggat ggatctagct 1260
caactagaga ggagtttatg gaggtgtgtt ttgtttattc actttgatta attagttgag 1320
aattttctta aaaacaattt ttgaatagtt ttataatatt cgttctttgg ttttgtctgc 1380
agaaatttaa gaatgctgtt tctaagggat tcgatcctga cgaacatctt gtgaaggttg 1440
gtattgcgaa tcagaccaca atgctcaagg gggaaaccga agagattggt tagtttttgc 1500
aactggttat gctatagatc ttgaggaact gccactactt gtttggaaca atgtttaaaa 1560
tgccgctttt atgcctcaag ggttttgtt tttgtgaggc ggggcgtacg cctcaaggcg 1620
ctttgttttt gtgaggcgag gcgtacgcct caaggcacat tgaggcataa acctcaaata 1680
tttaattaaa aaaatatttt tagaacacct aaagatataa agtaagatga atgtgtctta 1740
taaaactcaa acatagaata aaaaatgtca taaaactcaa acataccata aaatgtacca 1800
taaaactcaa acataaatat cttataaatt atcaaaattt tttaaataaa gttaactttt 1860
gtttctagaa ctagaatcct agattccact atcctagaat aaacttctga tcagtttttt 1920
tgggcctatg tatatcagtt atgttttaat ttgattttgg accttttgga cataaaaaac 1980
ccattacgaa aatacgcccc tgaggcgtac gctctctcaa tttccacctc agctgcccca 2040
actcagcgcc tcacctctgc taagcaaggt gcacgccttg ttgcgttttt tcccacctca 2100

aggcgcacct caaaaccgcc tttttaaaca ttggtttgga atgtattttg aacttgtatt 2160

cttaatttc agggaaactg gttgaacgcc ttggtgcgtt ttttttcgcc tcaaggcgca 2220

cctcaaaacc gcatttttaa acattggttt ggaatgtatt ttgaacttgt attcttaatt 2280

ttcagggaaa ctggttgaga gggctatgat gcaaaagtat ggagtggaaa acattaatga 2340

acacttccaa agctttaaca caatttgcga tgcgacccaa gtaataagtc ccttcctctt 2400

ctgtacatgt atccttgtct ggtttgttat ttctgagatt tcatttcact caaccgaaat 2460

ccttttcttt tccttgtttg ctcatataat agattgacga cattcctctt gtgtcatgta 2520

gtctgaacta atttttatg ttttgtcgtt tcccaggagc gtcaagatgc aatgtacaag 2580

ttggtggagg aacctattga tcttatgtta gttgttggag gatggaactc gagtaacacc 2640

tctcatctac aagagattgc agagaaacgt ggtattccct catattggat cgacagtgaa 2700

cagagaatag gtcctggaaa caaaatagcc tacaagctaa atgtaagttt aaacagatta 2760

ggctaccatt acaatctatt tatttctggt tttctgctaa tgtactagtt tagcagaact 2820

ccttagcaat gttttcttct tatctgtggt cctgattgtt gccaatttct tcttatgaat 2880

tttctgatag catggagagt tggttgagaa agagaactgg ttaccagagg gtcccatcac 2940

gattggtgta acatcaggtg cttctactcc agataaggtg agttcttatt cacaaaatcc 3000

ttaaaaacag caaaccacct caatgaaatc atgaaaagcc atgataaacc atattcttca 3060

ttctactcat aagacacaag gaagtgcaaa tttttctcat tgattttta tggttgtaca 3120

tctctctgct attttctgaa tcaggttgtt gaagatgttc tcatcaaggt gtttgacctt 3180

aaacgcgaag aagctttgca acttgcttag tttaagttag actcaatgtt tgtacatcac 3240

cagacagcgc tatcatatag cattattatt gtatttctag gatgaaatgt tgggtccttg 3300

ttatgcaaga gctccatgta tctaagaata ttaatcgtcg tatagtagta gaaatcacgt 3360

tgttattatt gaaagtgatc taataagttc tttgaatcta gatagcagcc atagcctgca 3420

ataatgcgtt taattgctga cacaataatg atattccacc 3460

<210> 5
<211> 3434
<212> DNA
<213> Humulus lupulus
<400> 5

```
actagtttct ctccacaatc tctgctattg tgcgcactcc ttcctactcc catgtcgatc     60
actttccacc tctgccggat cccaacccgt accgacctcg ccttgccgga ggccctctcc    120
agaaccggac ccttccgctg ccgaaaacct ttcgttgtcc gatgctccgg cgagtcatct    180
tccacggcag gagattccga cttcgatgcg aaagtgttcc gtaagaactt ggttcgaagc    240
aagaactaca atcggaaagg ttttggccat aaggaagaga ctcttcagct catgaacagc    300
```

gagtacacca gtatgctgtg cttcgattcc tcaatttgtt tataatgaat cgtaattatt 360
tagtttgatt aaataattaa tttactggaa aacaaaattc aggtgatatt ataaagactt 420
tgaaggataa cgggaacgag tatagctggg ggaacgtgac tgtgaaattg gccgaagctt 480
atgggttttg ctggggtgtg gagcgagctg tccagattgc ttacgaagca agaaaacagt 540
tccccgaaga aaaaatttgg attacaaacg aaatcattca caatcccaca gtcaacaagg 600
tttaatcttc attttatttt ttaattaatg ttagtgtttt cttcttcgta atggctcaat 660
ttatatattt attaatatta ttatgattat ttggatagag gctagaggaa atgaaagtag 720
aaaacattcc gattggtgaa gggaggaaac aattcgagat tgtaaacgag ggtgatgttg 780
tgatattgcc tgcgtttggt gctggagtgg atgagatgtt ggctttgagt gataggaatg 840
ttcaaattgt tgataccaca tgtccatggg tttccaaggt aaccttttc tatttttatt 900
tttttaataa aagattttta aaatatggga tattgtttat tatatgttat taatggtttt 960
gtttgcaaat gctctggttt gaaggtttgg aatacagtcg agaaacataa gaaggccgaa 1020
tacacttcca ttattcatgg taaatatgct catgaggaga ctattgcaac tgcatctttt 1080
gctggaactt acattattgt aaagaacatg aaagaggtaa caactttttc tcttatgaca 1140
aacaagtttt ttggtttatt ctttgtgctg tagattttga tggaatcttt attgtcttaa 1200
attcaggcaa tgtatgtgtg tgattacatt ctcggcggtc aactggatgg atctagctca 1260
actagagagg agtttatgga ggtgtgtttt gtttattcac tttgattaat tagttgacaa 1320
ttttcttaaa aacaattttt gaatagtttt ataatattcg ttctttggtt ttgtctgcag 1380
aaatttaaga atgctgtttc taagggattc gatcctgacg aacatcttgt gaaggttggt 1440
attgcgaatc agaccacaat gctcaagggg gaaaccgaag agattggtta gtttttgcaa 1500
ctggttatgc tatagatctt gaggaactgc cactacttgt ttggaacaat gtttaaaatg 1560
ccgcttttat gcctcaaggg gttttgtttt tgtgaggcga ggcgtacgcc tcaaggcaca 1620
ttgaggcata aacctcaaat atttaattaa aaaaatattt ttagaacacc taaagatata 1680
aagtaagatg aatgtgtcct ataaaactca aacatagaat aaaaaatgtc ataaaactca 1740
aacataccat aaaatgtaaa tgtaccataa aactcaaaca taaatatctt ataaattatc 1800
taaatttttt aaataaagta acttttgttt ctagaactag aatcctagat tccactatcc 1860
tagaataaac ttctgatcag ttttttgggg cctatttata tcagttgtgt tttaatttga 1920
tttggacctt ttggacataa aaaacccatt acgaaaatac gcccctgagg cgtacgctct 1980
ctcaatttcc acctcagctg tcccaactca gcgcctcact acagtgaggt gtatgcctca 2040
cctctgctaa gcaaggtgca cgccttggtg cgtttttttc cacctcaagg cgcacctcaa 2100
aactgccttt ttaaacattg gtttggaatg tattttgaac ttgtattctt aattttcagg 2160
gaaactggtt gaacgccttg gtgcgttttt ttgcgcctca aggcacacat caaaaccgca 2220

```
tttttaaaca ttggtttgga atgtattctt aattttcagg gaaactggtt gagagggcta   2280
tgatgcaaaa gtatggagtg gaaaacatta atgaacactt ccaaagcttt aacacaattt   2340
gcgatgcgac ccaagtaata agtcccttcc tcttctgtac atgtatcctt gtctggtttg   2400
ttatttctga gatttcattt cactcaaccg aaatcctttt cttttccttg tttgctcata   2460
taatagattg acgacattcc tcttgtgtca tgtagtctga actaattttt tatgttttgt   2520
cgtttcccag gagcgtcaag atgcaatgta caagttggtg gaggaaccta ttgatcttat   2580
gttagttgtt ggaggatgga actcgagtaa cacctctcat ctacaagaga ttgcagagga   2640
acgtggtatt ccctcgtatt ggatcgacag tgaacagaga ataggtcctg gaaacaaaat   2700
agcctacaag ctaaatgtaa gtttaaacag attaggctac cattacaatc tatttatttc   2760
tggttttctg tcaatgtact agtttagcag aactccttag caatgttttc ttcttatctg   2820
tggtcctgat tgttgtcaat ttcttcttat gaattttctg atagcatgga gagttggttg   2880
agaaagagaa ctggttacca gagggtccca tcacgattgg tgtaacatca ggtgcttcta   2940
ctccagataa ggtgagttct tattcacaaa atccttaaaa acagcaaacc acttcaatga   3000
aatcatgaaa agccatgata aaccatattc ttcattctac tcataagaca caaggaagtg   3060
caaatttttc tcattgattt tttatggttg tacatctctc tgctattttc tgaatcaggt   3120
tgttgaagat gttctcatca aggtgtttga ccttaaacgc gaagaagctt tgcaacttgc   3180
ttagtttaag ttagactcaa tgtttgtaca tcaccagaca gcgctatcat atagcattat   3240
tattgtattt ctaggatgaa atgttgggtc cttgttatgc aagagctcca tgtatctaag   3300
aatattaatc gtcgtatagt agtagaaatc acgttgttat tattgaaagg gatctaataa   3360
gttctttgaa tctagatagc agccatagcc tgcaataatg cgtttaattg ctgacacaat   3420
aatgatattc cacc                                                     3434
```

<210> 6
<211> 3460
<212> DNA
<213> Humulus lupulus
<400> 6

actagtttct ctccacaatc tctgctattg tgcgcactcc ttcctactcc catgtcgatc 60

actttccacc tctgccggat cccaacccgt accgacctcg ccttgccgga ggccctctcc 120

agaaccggac ccttccgctg ccgaaaacct ttcgttgtcc gatgctccgg cgagtcatct 180

tccacggcag gagattccga cttcgatgcg aaagtgttcc gcaagaactt ggttcgaagc 240

aagaactaca atcggaaagg ttttggccat aaggaagaga ctcttcagct catgaacagc 300

gagtacacca gtatgctgtg cttcgattcc tcaatttgtt tataatgaat cgtaattatt 360

tagtttgatt aaataattaa tttactggaa aacaaaattc aggtgatatt ataaagactt 420

tgaaggataa cgggaacgag tatagctggg ggaacgtgac tgtgaaattg gccgaagctt 480
atgggttttg ctggggtgtg gagcgagctg tccagattgc ttacgaagca agaaaacagt 540
tccccgaaga aaaaatttgg attacaaacg aaatcattca caatcccaca gtcaacaagg 600
tttaatcttc attttatttt ttgattaatg ttagtgtttt cttcttcgta atggctcaat 660
ttatatattt attaatatta ttatgattat ttggatagag gctagaggaa atgaaagtag 720
aaaacattcc gattggtgaa gggaggaaac aattcgagat tgtaaacgag ggtgatgttg 780
tgatattgcc tgcgtttggt gctggagtgg atgagatgtt ggctttgagt gataggaatg 840
ttcaaattgt tgataccaca tgtccatggg tttccaaggt aacctttttc tatttttatt 900
tttttaataa aagattttta aaatatggga tattgtttaa attatatgtt attaatggtt 960
ttggttgcaa atgctctggt ttgaaggttt ggaatacagt cgagaaacat aagaaggccg 1020
aatacacttc cattattcat ggtaaatatg ctcatgagga gactattgca actgcatctt 1080
ttgctggaac ttacattatt gtaaagaaca tgaaagaggt aacaactttt tctcttatga 1140
caaacaagtt ttttggttta ttctttgtgc tgtagatttt gatggaatct ttattgtctt 1200
aaattcaggc agtgtatgtg tgtgattaca ttctcggcgg tcaactggat ggatctagct 1260
caactagaga ggagtttatg gaggtgtgtt ttgtttattc actttgatta attagttgag 1320
aattttctta aaaacaattt ttgaatagtt ttataatatt cgttctttgg ttttgtctgc 1380
agaaatttaa gaatgctgtt tctaagggat tcgatcctga cgaacatctt gtgaaggttg 1440
gtattgcgaa tcagaccaca atgctcaagg gggaaaccga agagattggt tagtttttgc 1500
aactggttat gctatagatc ttgaggaact gccactactt gtttggaaca atgtttaaaa 1560
tgccgctttt atgcctcaag ggttttgtt tttgtgaggc ggggcgtacg cctcaaggcg 1620
ctttgttttt gtgaggcgag gcgtacgcct caaggcacat tgaggcataa acctcaaata 1680
tttaattaaa aaaatatttt tagaacacct aaagatataa agtaagatga atgtgtctta 1740
taaaactcaa acatagaata aaaaatgtca taaaactcaa acataccata aaatgtacca 1800
taaaactcaa acataaatat cttataaatt atcaaaattt tttaaataaa gttaactttt 1860
gtttctagaa ctacaatcct agattccact atcctagaat aaacttctga tcagtttttt 1920
tgggcctatg tatatcagtt atgttttaat ttgattttgg accttttgga cataaaaaac 1980
ccattacgaa aatacgcccc tgaggcgtac gctctctcaa tttccacctc agctgcccca 2040
actcagcgcc tcacctctgc taagcaaggt gcacgccttg ttgcgttttt tcccacctca 2100
aggcgcacct caaaaccgcc tttttaaaca ttggtttgga atgtattttg aacttgtatt 2160
cttaattttc agggaaactg gttgaacgcc ttggtgcgtt ttttttcgcc tcaaggcgca 2220
cctcaaaacc gcatttttaa acattggttt ggaatgtatt ttgaacttgt attcttaatt 2280

ttcagggaaa ctggttgaga gggctatgat gcaaaagtat ggagtggaaa acattaatga 2340
acacttccaa agctttaaca caatttgcga tgcgacccaa gtaataagtc ccttcctctt 2400
ctgtacatgt atccttgtct ggtttgttat ttctgagatt tcatttcact caaccgaaat 2460
ccttttcttt tccttgtttg ctcatataat agattgacga cattcctctt gtgtcatgta 2520
gtctgaacta attttttatg ttttgtcgtt tcccaggagc gtcaagatgc aatgtacaag 2580
ttggtggagg aacctattga tcttatgtta gttgttggag gatggaactc gagtaacacc 2640
tctcatctac aagagattgc agaggaacgt ggtattccct catattggat cgacagtgaa 2700
cagagaatag gtcctggaaa caaaatagcc tacaagctaa atgtaagttt aaacagatta 2760
ggctaccatt acaatctatt tatttctggt tttctgctaa tgtactagtt tagcagaact 2820
ccttagcaat gttttcttct tatctgtggt cctgattgtt gccaatttct tcttatgaat 2880
tttctgatag catggagagt tggttgagaa agagaactgg ttaccagagg gtcccatcac 2940
gattggtgta acatcaggtg cttctactcc agataaggtg agttcttatt cacaaaatcc 3000
ttaaaaacag caaaccacct caatgaaatc atgaaaagcc atgataaacc atattcttca 3060
ttctactcat aagacacaag gaagtgcaaa tttttctcat tgattttta tggttgtaca 3120
tctctctgct attttctgaa tcaggttgtt gaagatgttc tcatcaaggt gtttgacctt 3180
aaacgcgaag aagctttgca acttgcttag tttaagttag actcaatgtt tgtacatcac 3240
cagacagcgc tatcatatag cattattatt gtatttctag gatgaaatgt tgggtccttg 3300
ttatgcaaga gctccatgta tctaagaata ttaatcgtcg tatagtagta gaaatcacgt 3360
tgttattatt gaaagtgatc taataagttc tttgaatcta gatagcagcc atagcctgca 3420
ataatgcgtt taattgctga cacaataatg atattccacc 3460

<210> 7
<211> 20
<212> DNA
<213> Artificial
<220>
<221> source
<223> /note="Description of artificial sequence: primer"
<400> 7
tgctgtttct aagggattcg 20
<210> 8
<211> 20
<212> DNA
<213> Artificial
<220>
<221> source
<223> /note="Description of artificial sequence: primer"
<400> 8
tttccaggac ctattctctg 20

## Description

### Technical Field

The present invention relates to a method for screening of hop varieties and to a breeding marker and primer set to be used for screening of hop varieties.

### Background Art

Hops, the primary material for beer, impart a refreshing bitterness and aroma to beer. The known bittering agents in hops include a acids such as humulone, cohumulone and adhumulone, and β acids such as lupulone, colupulone and adlupulone. The α acids exhibit antibacterial activity, anticancer activity, anti-inflammatory effects and so on, and the β acids exhibit sedative effects and so on, and thus, they are both being considered also as drug sources (Non-patent document 1).

The α acid contained in hops, when it is treated with heat during a beer production process, is isomerized into iso-α acid (iso-humulone, iso-cohumulone and iso-adhumulone). It is known that when iso-humulone and iso-cohumulone (which occupy a major part of iso α acids) are compared, iso-cohumulone is inferior in sensuality, more specifically, the quality of bitterness is low, and also inferior in beer-foam stabilization (Non-Patent Document 2). Since iso-cohumulone is produced by isomerization of cohumulone, it is desired to screen for hop varieties having a low ratio of the cohumulone contents (herein after, cohumulone ratio) relative to the contents of the α acid contents in hops, in breeding hops.

In the β-acids contained in hops, lupulone and colupulone are mostly included. It is known that degree of bitterness can be reduced (milder) or increased (stronger) depending upon the contents of β-acids in hops (Non-Patent Document 3).

It is kwon that farnesene is a component detected only in the top-grade hops called fine aroma hop, whereas linalool imparts rose-like flower scent to beer (Non-Patent Document 2). Farnesene and linalool contained in hops are considered as an important component for characterizing beer in breeding hops.

Methods used for breeding of useful plants include methods in which high-grade varieties are crossed and the obtained hybrid progeny are cultivated until expression of the desired traits and screened, and molecular screening methods in which the genomic DNA of hybrid progeny are examined at the seed or seedling stage and screened based on breeding markers (DNA markers).

[Non-Patent Document 1] Gerhaeuser, European Journal of Cancer, 2005, Vol. 41, No. 13, p.1941-195.4
[Non-Patent Document 2] Peacock et al., European Brewing Convention monograph XXII Symposium on hops Zoeterwoude, 1994, p.247-250
[Non-Patent Document 3] Kowaka et al., EBC proceedings, 1983, Vol. 19, p.71-78
[Non-Patent Document 4] Peacock et al., J. Agric. Food Chem., 1980, Vol. 28, p.774-777

### Disclosure of the Invention

### Problems to be Solved by the Invention

However, virtually no genetic research has been conducted on male hop plants, which produce no cones, and virtually none of the genes involved in biosynthesis of the aforementioned components associated with hop bitterness and aroma have been identified even in female plants. Consequently, it has not been possible to utilize molecular screening methods based on breeding markers, for screening of hop varieties based on ratio of cohumulone and/or colupulone and contents of farnesene and/or linalool.

In addition, since α acids, β acids, farnesene and linalool accumulate specifically in the lupulin gland of hop cones, screening of hop varieties with high levels of these components can only be done after seeding and waiting for forming of the cones, and at least several years of cultivation have been necessary for stabilization of these traits.

It is therefore an object of the present invention to utilize a breeding marker-based molecular screening method for screening of hop varieties with low cohumulone ratio, within a shorter time period. It is another object of the invention to screen for hop varieties with low ratio of cohumulone and colupulone, and with high contents of farnesene and/or linalool, within a shorter time period.

### Means for Solving the Problems

In order to achieve the objects stated above, the invention provides breeding markers, represented by any one of the following (a) to (d), to be used for screening of hop varieties with low ratio of cohumulone contents relative to α acid contents (cohumulone ratio) in hops.

(a) A polynucleotide consisting of a nucleotide sequence of 20-3434 continuous nucleotides including nucleotide No.623 of the polynucleotide consisting of the nucleotide sequence as set forth in SEQ ID NO: 5 (wherein the 623th nucleotide has adenine).

(b) A polynucleotide consisting of the sequence complementary to the polynucleotide of (a) above.

(c) A polynucleotide consisting of a nucleotide sequence of 20-3434 continuous nucleotides including nucleotide No.1820 of the polynucleotide consisting of the nucleotide sequence as set forth in SEQ ID NO: 5 (wherein the 1820th nucleotide has adenine).

(d) A polynucleotide consisting of the sequence complementary to the polynucleotide of (c) above.

The present inventors have discovered the 623rd nucleotide (having adenine) and the 1820th nucleotide (having adenine) of the polynucleotide consisting of the nucleotide sequence as set forth in SEQ ID NO: 5, as a polymorphism found to be common to hop varieties with low cohumulone ratio, from a cDNA library prepared from the lupulin gland of hop cones. No breeding marker for anticipation of low cohumulone ratio that can be applied to all hops has been reported to date, but it was found that this breeding marker allows screening of hop varieties with low cohumulone ratio among hybrid progeny obtained by crossing various hop lines. Also, it was demonstrated that the hop varieties screened with the breeding marker also have low colupulone ratio and high contents of farnesene and/or linalool.

If, during breeding of hop varieties, those hop varieties having the same sequence as the breeding marker are selected, it is possible to screen for hop varieties with low cohumulone ratio at the seed or seedling stage without waiting for the hops to grow and form cones. Moreover, determining the presence of the breeding marker allows objective, genomic information-based identification of the combinations of crossed lines, that has hitherto depended on intuition and experience. The breeding marker may also be suitably used for screening of hop varieties with low colupulone ratio and with high contents of farnesene and/or linalool.

Here, the phrase "hop varieties with low cohumulone ratio" refers to hop varieties in groups with low average values for cohumulone ratio, when groups of hybrid progeny are categorized according to whether or not they have the same sequence as the aforementioned breeding marker. The phrase "hop varieties with low colupulone ratio" refers to hop varieties in groups with low average values for colupulone ratio, when groups of hybrid progeny are categorized according to whether or not they have the same sequence as the aforementioned breeding marker. The phrase "hop varieties with high contents of farnesene and/or linalool" refers to hop varieties in groups with high average values for contents of farnesene and/or linalool, when groups of hybrid progeny are categorized according to whether or not they have the same sequence as the aforementioned breeding marker.

The invention still further provides a screening method for hop varieties with low ratio of cohumulone contents relative to $\alpha$ acid contents (low cohumulone ratio) in hops, comprising: an extraction step in which genomic DNA is extracted from a hop variety specimen; a digestion step in which the genomic DNA is digested with restriction enzyme XbaI to obtain digested genomic DNA fragments; a detection step in which the digested genomic DNA fragments are separated and genomic DNA fragments that hybridize to any one of the polynucleotide of (a) to (d) above are detected; And a judging step in which it is judged that the variety has low cohumulone ratio when a genomic DNA fragment of about 8.6 kbp is not detected in the detection step above.

With this screening method it is possible to screen for hop varieties with low cohumulone ratio, hop varieties with low colupulone ratio, and hop varieties with high contens of farnesene and/or linalool, by using any one of the polynucleotide of (a) to (d) as a probe and examining Restriction Fragment Length Polymorphisms (RFLP) with restriction enzyme XbaI. Since genome fragment sizes can be visually discriminated, it is possible to objectively and rapidly screen for hop varieties having these features without examining the nucleotide sequences of the genomic DNA.

The invention yet further provides a method for screening of hop varieties with low ratio of cohumulone contents relative to $\alpha$ acid contents (low cohumulone ratio) in hops, which comprises an extraction step in which genomic DNA is extracted from a hop variety specimen, and an identifying step in which genomic DNA polymorphism at the 623rd nucleotide position of the nucleotide sequence as set forth in SEQ ID NO: 5 is identified.

The identifying step preferably comprises a PCR step in which DNA is synthesized by PCR using the aforementioned genomic DNA as template, a primer consisting of a nucleotide sequence of 18-50 contiguous nucleotides located toward the 5'-end from the 623rd nucleotide of the nucleotide sequence as set forth in SEQ ID NO: 5 and a primer consisting of the sequence complementary to a nucleotide sequence of 18-50 contiguous nucleotides located toward the 3'-end from the 623rd nucleotide of the nucleotide sequence as set forth in SEQ ID NO: 5, a digestion step in which the DNA is digested with restriction enzyme PacI, and a judging step in which the variety is judged to have low cohumulone ratio when the DNA is not cleaved with restriction enzyme PacI in the digestion step above.

In this screening method, PCR is performed using the extracted genomic DNA as a template and whether or not the amplified DNA is cleaved with restriction enzyme PacI is used as an index. Therefore no Southern hybridization is necessary. If the sizes of the DNA fragments after the digestion step are confirmed based on, for example, fractionation by agarose electrophoresis, whether or not the synthesized DNA in the PCR step was cleaved by restriction enzyme PacI can be easily determined, thus allowing screening of hop varieties with low cohumulone ratio or hop varieties with low colupulone ratio as well as low cohumulone ratio, and hop varieties with high contents of farnesene and/or linalool.

The present invention also provides a method for screening of hop varieties with low ratio of cohumulone contents relative to α acid contents (cohumulone ratio) in hops which comprises: an extraction step in which genomic DNA is extracted from a hop variety specimen; and an identifying step in which genomic DNA polymorphism at the 1820th nucleotide position of the nucleotide sequence as set forth in SEQ ID NO: 5 is identified.

It is preferred that the identifying step comprises: a PCR step in which DNA is synthesized by PCR using the aforementioned genomic DNA as template, a primer consisting of a nucleotide sequence of 18-50 contiguous nucleotides located toward the 5'-end from the 1820th nucleotide of the nucleotide sequence as set forth in SEQ ID NO: 5 and a primer consisting of the sequence complementary to a nucleotide sequence of 18-50 contiguous nucleotides located toward the 3'-end from the 1820th nucleotide of the nucleotide sequence as set forth in SEQ ID NO: 5; a digestion step in which the DNA is digested with restriction enzyme HpaI; and a judging step in which the variety is judged to have low cohumulone ratio when all kinds of the DNA are cleaved with restriction enzyme HpaI in the digestion step above.

In this screening method, PCR is performed using the extracted genomic DNA as a template and whether or not the amplified DNA is cleaved with restriction enzyme HpaI is used as an index. Therefore no Southern hybridization is necessary. If the sizes of the DNA fragments after the digestion step are confirmed based on, for example, fractionation by agarose electrophoresis, whether or not the synthesized DNA in the PCR step was cleaved by restriction enzyme HpaI can be easily determined, thus allowing screening of hop varieties with low cohumulone ratio or hop varieties with low colupulone ratio as well as low cohumulone ratio, and hop varieties with high contents of farnesene and/or linalool.

It is preferred that the identifying step comprises: a PCR step in which DNA is synthesized by PCR using the aforementioned genomic DNA as template, a primer including the nucleotide sequence as set forth in SEQ ID NO: 7 and a primer including the nucleotide sequence as set forth in SEQ ID NO: 8; a digestion step in which the DNA is digested with restriction enzyme HpaI; and a judging step in which the variety is judged to have low cohumulone ratio when a DNA fragment of about 1304 bp is not contained in the DNA fragments obtained by the digestion step above.

In this screening method, since PCR is conducted using the extracted genomic DNA as template, a primer containing the nucleotide sequence as set forth in SEQ ID NO: 7 and a primer containing the nucleotide sequence as set forth in SEQ ID NO: 8, only the target DNA fragment is efficiently and specifically amplified. In addition, if the sizes of the DNA fragments obtained by the digestion step are confirmed based on, for example, fractionation by agarose electrophoresis, whether or not an approximately 1304 bp fragment is present can be easily determined, thus allowing screening of hop varieties with low cohumulone ratio or hop varieties with low colupulone ratio as well as low cohumulone ratio, and hop varieties with high contents of farnesene and/or linalool.

The present invention also provides a primer set to be used for screening of hop varieties with low ratio of cohumulone contents relative to α acid contents (cohumulone ratio) in hops, the primer set comprising: a primer containing the nucleotide sequence as set forth in SEQ ID NO: 7; And a primer containing the nucleotide sequence as set forth in SEQ ID NO: 8.

Using this primer set allows specific amplification of only the DNA fragment containing the 1820th nucleotide of the nucleotide sequence as set forth in SEQ ID NO: 5, using the extracted genomic DNA as template. The primer set can be appropriately utilized for screening of hop varieties with low cohumulone ratio or hop varieties with low colupulone ratio as well as low cohumulone ratio, and hop varieties with high contents of farnesene and/or linalool.

## Effect of the Invention

According to the invention it is possible to provide a breeding marker for hop varieties with low cohumulone ratio or hop varieties with low colupulone ratio as well as low cohumulone ratio, and hop varieties with high contents of farnesene and/or linalool. According to the invention it is also possible to objectively and rapidly screen for hop varieties with low cohumulone ratio or hop varieties with low colupulone ratio as well as low cohumulone ratio, and hop varieties with high contents of farnesene and/or linalool, using hop seeds or seedlings, without waiting for the hops to form cones and develop stabilized traits.

## Brief Description of the Drawings

Fig. 1 is an image showing XbaI/3D03 polymorphism, obtained by digestion of genomic DNA of hop varieties with restriction enzyme XbaI, and genomic Southern hybridization.

Fig. 2 shows a single nucleotide polymorphism obtained by analysis of homology of the nucleotide sequences of e-type g3D03, f-type g3D03 and g-type g3D03m.

Fig. 3 shows a typical band pattern for HpaI/g3D03 polymorphism, obtained by PCR-RFLP of hop varieties.

Fig. 4 is an image showing HpaI/g3D03 polymorphism, obtained by analysis of genomic DNA of 9 hop lines.

Fig. 5 is an image showing XbaI/3D03 polymorphism, obtained by genomic analysis of 9 hop lines.

## Best Modes for Carrying Out the Invention

Preferred embodiments of the invention will now be described in detail.

The breeding markers of the invention are characterized by being represented by any one of the following (a) to (d), which is used for screening of hop varieties with low ratio of cohumulone contents relative to α acid contents (cohumulone ratio) in hops.

(a) A polynucleotide consisting of a nucleotide sequence of 20-3434 continuous nucleotides including nucleotide No.623 of the polynucleotide consisting of the nucleotide sequence as set forth in SEQ ID NO: 5 (wherein the 623th nucleotide has adenine).

(b) A polynucleotide consisting of the sequence complementary to the polynucleotide of (a) above.

(c) A polynucleotide consisting of a nucleotide sequence of 20-3434 continuous nucleotides including nucleotide No.1820 of the polynucleotide consisting of the nucleotide sequence as set forth in SEQ ID NO: 5 (wherein the 1820th nucleotide has adenine).

(d) A polynucleotide consisting of the sequence complementary to the polynucleotide of (c) above.

The term "breeding marker" means a DNA marker used for breeding, and it is a DNA sequence that exists in a genome near a gene associated with a trait of interest and operates together with the gene. A breeding marker allows identification of traits that cannot be distinguished by the naked eye, such as disease resistance, or traits that appear in the mature phase, by using genomic DNA from seeds or seedlings of the plant specimen. The breeding marker may be the gene itself, or DNA other than the gene.

The term "cohumulone ratio" means the ratio of cohumulone contents relative to the contents of α acid in hops. The term "colupulone ratio" means the ratio of the colupulone contents relative to the contents of β acid in hops. The term "a acid" refers to a bitter substance in hops that is primarily humulone and its analogs including cohumulone, adhumulone and so on. The term "β acid" refers to a bitter substance in hops that is primarily lupulone and its analogs including colupulone, adlupulone and so on.

Throughout the present specification, "hops" refers to the perennial plant (Humulus lupulus L.) itself, and "cones" refers to the female flower produced by female hops, where the perichaetium has formed a pine cone-shaped chamber. The term "hop variety" means each hybrid progeny obtained by breeding hops.

The term "polynucleotide" refers to a molecule with a plurality of bonded phosphoric acid esters of nucleosides comprising purine or pyrimidine bases that are β-N-glycoside bonded to sugars (ribonucleotides or deoxyribonucleotides).

The polynucleotides represented by (a) to (d) above may be cloned from a hop genomic library using a labeled DNA probe synthesized based on the nucleotide sequence information of SEQ ID NO: 5, according to the method described in, for example, Molecular cloning (Maniatis et al., 1989, Cold Spring Harbor Laboratory Press).

The polynucleotides represented by (a) to (d) may also be synthesized by PCR using a primer set designed based on the nucleotide sequence information of SEQ ID NO: 5, with genomic DNA extracted from tissue of hop leaves or the like as template. Also, partial sequences of the polynucleotide represented by (a) to (d) may be synthesized with a DNA synthesizer and linked by an enzymatic method and subcloning, to obtain a polynucleotide of the target size.

The breeding marker may be used for screening of hop varieties with low cohumulone ratio, but it is preferably used for screening of hop varieties with low colupulone ratio as well as low cohumulone ratio, and hop varieties with high contents of farnesene and/or linalool.

Also, the screening method according to the invention, which is for hop varieties with low ratio of cohumulone contents relative to α acid contents in hops, is characterized by comprising: an extraction step in which genomic DNA is extracted from a hop variety specimen; a digestion step in which the genomic DNA is digested with restriction enzyme XbaI to obtain digested genomic DNA fragments; a detection step in which the digested genomic DNA fragments are separated and genomic DNA fragments that hybridize to any one of the polynucleotide of (a) to (d) above are detected; anda judging step in which it is judged that the variety has low cohumulone ratio when a genomic DNA fragment of about 8.6 kbp is not detected in the detection step above.

Methods utilizing RFLP may be mentioned as examples of such screening methods. Specifically, this method compares the sizes of DNA fragments produced after treatment with a restriction enzyme, based on mutations or nucleotide insertions or deletions at a restriction enzyme recognition site in the genomic DNA, to determine whether or not a certain trait is present.

In the detection step, it is possible to detect genomic DNA fragments that hybridize to the polynucleotides represented by (a) to (d) by genomic Southern hybridization, as described in Molecular cloning (Maniatis et al., 1989, Cold Spring Harbor Laboratory Press), for example. Specifically, the digested genomic DNA fragments of a hop specimen digested with restriction enzyme XbaI are fractionated by agarose gel electrophoresis, the fractionated genomic DNA fragments are transferred to a nylon membrane, a labeled polynucleotide represented by (a) to (d) above is used as a probe for hybridization, and the hybridized bands are detected based on the probe labeling.

The probe labeling method may be a method in which a DNA polymerase such as Klenow enzyme is used to incorporate substrate nucleotides that are labeled with an isotope such as $^{32}P$, a fluorescent dye, digoxigenin (DIG) or biotin, using random hexamer oligonucleotides and the like as primer (random primer method), or a method in which T4 polynucleotide kinase is used for phosphorylation of the 5'-end of the oligonucleotide with $^{32}P$ or the like for labeling.

In the judging step, digested genomic DNA fragments from a hop variety specimen may be electrophoresed with a DNA size marker and transferred to a nylon membrane, with the well positions and size marker migration positions marked on the nylon membrane, thus allowing the sizes of the digested genomic DNA fragments from hybridized hop variety specimens to be calculated based on the relationship between migration distance from the well and DNA size marker size. As a result, if a digested genomic DNA fragment of approximately 8.6 kbp is not detected, the hop variety specimen may be judged to be a variety with low cohumulone ratio.

Also, the screening method according to the invention, which is for hop varieties with low ratio of cohumulone contents relative to $\alpha$ acid contents in hops, is characterized by comprising: an extraction step in which genomic DNA is extracted from a hop variety specimen; and an identifying step in which genomic DNA polymorphism at the 623rd nucleotide position of the nucleotide sequence as set forth in SEQ ID NO: 5 is identified.

The identifying step preferably comprises: a PCR step in which DNA is synthesized by PCR using the aforementioned genomic DNA as template, a primer consisting of a nucleotide sequence of 18-50 contiguous nucleotides located toward the 5'-end from the 623rd nucleotide of the nucleotide sequence as set forth in SEQ ID NO: 5 and a primer consisting of the sequence complementary to a nucleotide sequence of 18-50 contiguous nucleotides located toward the 3'-end from the 623rd nucleotide of the nucleotide sequence as set forth in SEQ ID NO: 5; a digestion step in which the DNA is digested with restriction enzyme PacI; and a judging step in which the variety is judged to have low cohumulone ratio when the DNA is not cleaved with restriction enzyme PacI in the digestion step above.

Methods utilizing PCR-RFLP may be mentioned as examples of such a screening method. Specifically, such methods compare the sizes of DNA fragments produced after treatment with a restriction enzyme, based on mutations or nucleotide insertions or deletions at a restriction enzyme recognition site in the genomic DNA or a DNA fragment amplified by PCR using cDNA as template, to determine whether or not a certain trait is present.

The primer used in the PCR step may be synthesized with a DNA synthesizer, selecting a nucleotide sequence of 18-50 contiguous nucleotides from the nucleotide sequence as set forth in SEQ ID NO: 5. The size of the primer is preferably at least 18 and no greater than 30 nucleotides, and more preferably at least 20 and no greater than 22 nucleotides.

In the judging step, DNA fragments from a hop variety specimen digested with restriction enzyme PacI may be fractionated by agarose electrophoresis or polyacrylamide electrophoresis together with a DNA size marker, thus allowing calculation of the sizes of the digested DNA fragments from hop variety specimens based on the relationship between migration distance of the DNA size marker from the well and the sizes of the DNA size marker. If the sizes of the DNA fragments from the hop variety specimen are known, then it is possible to easily judge whether or not the synthesized DNA in the PCR step was cleaved by restriction enzyme PacI, so that the hop variety specimen can be judged as a variety with low cohumulone ratio when cleavage has been confirmed. The digested DNA fragments and size marker in the gel after electrophoresis may be detected under UV irradiation after treatment with, for example, ethidium bromide or SYBR Green.

Furthermore, the screening method of the present invention is a method for screening of hop varieties with low ratio of cohumulone contents relative to $\alpha$ acid contents in hops (cohumulone ratio) which comprises: an extraction step in which genomic DNA is extracted from a hop variety specimen; and an identifying step in which genomic DNA polymorphism at the 1820th nucleotide position of the nucleotide sequence as set forth in SEQ ID NO: 5 is identified.

It is preferred that the identifying step comprises: a PCR step in which DNA is synthesized by PCR using the aforementioned genomic DNA as template, a primer consisting of a nucleotide sequence of 18-50 contiguous nucleotides located toward the 5'-end from the 1820th nucleotide of the nucleotide sequence as set forth in SEQ ID NO: 5 and a primer consisting of the sequence complementary to a nucleotide sequence of 18-50 contiguous nucleotides located toward the 3'-end from the 1820th nucleotide of the nucleotide sequence as set forth in SEQ ID NO: 5; a digestion step in which the DNA is digested with restriction enzyme HpaI; and a judging step in which the variety is judged to have low cohumulone ratio when all kinds of the DNA are cleaved with restriction enzyme HpaI in the digestion step above.

As such a method, for example, a PCR-RFLP method may be mentioned. The specific procedure of the method and synthesis of primers are as mentioned above. The size of primers are preferably not less than 18 and not more than 30 bases, and further preferably not less than 20 and not more than 22 bases.

In the judging step, DNA fragments from a hop variety specimen digested with restriction enzyme HpaI may be fractionated by agarose electrophoresis or polyacrylamide electrophoresis together with a DNA size marker, thus allowing calculation of the sizes of the digested DNA fragments from hop variety specimens based on the relationship between migration distance of the DNA size marker from the well and the sizes of the DNA size marker. If the sizes of the DNA fragments from the hop variety specimen are known, then it is possible to easily judge whether or not the synthesized DNA in the PCR step was cleaved by restriction enzyme HpaI, so that the hop variety specimen can be judged as a variety with low cohumulone ratio when cleavage has been con&med. The digested DNA fragments and size marker in the gel after electrophoresis may be detected under UV irradiation after treatment with, for example, ethidium bromide or SYBR Green.

It is preferred that the identifying step comprises: a PCR step in which DNA is synthesized by PCR using the aforementioned genomic DNA as template, a primer including the nucleotide sequence as set forth in SEQ ID NO: 7 and a primer including the nucleotide sequence as set forth in SEQ ID NO: 8; a digestion step in which the DNA is digested with restriction enzyme HpaI; and a judging step in which the variety is judged to have low cohumulone ratio when a DNA fragment of about 1304 bp is not contained in the DNA fragments obtained by the digestion step above.

The primers to be used in the aforementioned PCR step can be synthesized by a DNA synthesizer separately based on the nucleotide sequence as set forth in SEQ ID NO: 7 and the nucleotide sequence as set forth in SEQ ID NO: 8.

In the judging step, DNA fragments from a hop variety specimen digested with restriction enzyme HpaI may be fractionated by agarose electrophoresis or polyacrylamide electrophoresis together with a DNA size marker, thus allowing calculation of the sizes of the digested DNA fragments from hop variety specimens based on the relationship between migration distance of the DNA size marker from the well and the sizes of the DNA size marker. As a result, if the size of at least one type of DNA fragment derived from the hop variety specimen is not detected in the proximity of a size of 1304 bp, the hop variety specimen can be judged as a variety having low cohumulone ratio. The digested DNA fragments and size marker in the gel after electrophoresis may be detected under UV irradiation after treatment with, for example, ethidium bromide or SYBR Green.

The screening method of the invention may be used for screening of hop varieties with low cohumulone ratio, but it is preferably used for screening of hop varieties with low colupulone ratio, and hop varieties with high contents of farnesene and/or linalool, as well as hop varieties with low cohumulone ratio.

The primer set of the present invention is a primer set to be used for screening of hop varieties with low ratio of cohumulone contents relative to α acid contents in hops, the primer set comprising: a primer containing the nucleotide sequence as set forth in SEQ ID NO: 7; And a primer containing the nucleotide sequence as set forth in SEQ ID NO: 8.

The primer set may be synthesized with a DNA synthesizer based on the nucleotide sequence as set forth in SEQ ID NO: 7 and the nucleotide sequence as set forth in SEQ ID NO: 8.

## Examples

The present invention will now be explained in greater detail with reference to examples, with the understanding that the invention is not meant to be limited to these examples.

1. Experiment method

1) Quantitation of β acids in hop cones:

The quantities of β acids in the hop cones were measured by a modified mode of the official method of the American Society of Brewing Chemists (ASBC). Specifically, 1 g of pulverized hop cones was first placed in a 100 mL stoppered Erlenmeyer flask, and then 4 mL of methanol and 20 mL of diethyl ether were added prior to shaking for 30 minutes. Next, 8 mL of 0.1 M hydrochloric acid was added prior to shaking for 10 minutes, and after standing, 0.2 mL of the ether layer was sampled, methanol was added to a volume of 10 mL and a portion was analyzed by high performance liquid chromatography (HPLC). The HPLC conditions were based on Official Method 7.7 of the European Brewery Convention (EBC), and the α acids, β acids were detected and quantified at a wavelength of 314 nm.

The cohumulone ratio and colupulone ratio were calculated in comparison with the amounts of humulone, cohumulone, lupulone and colupulone of the reference sample of a bittering agent of hop, namely, ICE-2 (manufactured by Labor Veritas AG).

2) Quantitation of farnesene and linalool in hop cones:

The quantity of farnesene and linalool in hop cones was determined by the following procedure. First, 1 g of pulverized hop cones was placed in a 10 mL stoppered centrifuge tube, and then 6 mL of n-hexane was added prior to shaking for 45 minutes, after which the mixture was centrifuged at 3,000 rpm, 20°C for 10 minutes. Next, 1.5 mL of the supernatant was sampled in a 10 mL stoppered centrifuge tube, and 3 mL of 5% potassium carbonate was added prior to shaking for 5 minutes, after which the mixture was centrifuged at 3,000 rpm, 20°C for 10 minutes and the supernatant was analyzed by gas chromatography (HP5890; manufactured by Hewlett-Packard Company). As the column, a J&W capillary column (DB-1, 0.25 mm × 30 cm × 1.0 μm; manufactured by GL Sciences Inc.) was used. As reference samples for farnesene and linalool, (E)-β-farnesene (manufactured by Wako Pure Chemical Industries Ltd.) and (±)-Linalool (manufactured by SIGMA) were used.

3) Preparation of total RNA and poly $A^+$ RNA from lupulin gland of hop cones:

First, the frozen cones of female hop plants were crushed and the fraction that passed through a 250 μm mesh diameter sieve was used as the lupulin gland fraction. The lupulin gland fraction was then homogenized in liquid nitrogen, a 2% CTAB solution (2% cetyltrimethylammonium bromide, 20 mM EDTA, 1.4 M NaCl, 5% β-mercaptoethanol, 0.1 M Tris, pH 9.5) was added to create a suspension, and after warming at 65°C for 10 minutes, extraction was performed twice with chloroform/isoamyl alcohol (24:1). A 1/3-fold amount of 10 M lithium chloride was added to the obtained water-soluble fraction, and the mixture was allowed to stand overnight and then centrifuged at 15,000 rpm for 10 minutes, after which the obtained precipitate was dissolved in RNase-free sterilized water. Next, a 1/3-fold amount of 10 M lithium chloride was further added to the resulting solution, and the mixture was allowed to stand overnight and then centrifuged at 15,000 rpm for 10 minutes, after which the obtained precipitate was dissolved in RNase-free sterilized water for use as the total RNA sample.

The obtained total RNA sample was quantitated by measurement of the absorbance at 260 nm using a spectrophotometer, and then Oligotex-dT30 Super by Takara Bio, Inc. was used for preparation of poly $A^+$ RNA following the manufacturer's protocol.

4) Preparation of hop cone lupulin gland cDNA library:

A Creator SMART cDNA Library Construction Kit by BD Biosciences was used to prepare a hop cone lupulin gland cDNA library from the poly $A^+$ RNA obtained from hop cone lupulin gland. The procedure for preparation of the cDNA library was according to the manufacturer's protocol supplied with the kit.

2. Experimental results

(Example 1) Search for breeding markers related to cohumulone ratio, colupulone ratio, farnesene contents and linalool contents.

First, clones were randomly selected from a hop cone (Saaz variety) lupulin gland cDNA library, and lupulin gland cDNA introduced into each clone was used as template for PCR in the presence of digoxigenin (DIG)-labeled dUTP, to create DIG-labeled probes. The DIG-labeled probes were created by PCR using a PCR DIG probe synthesis kit by Roche Diagnostics K.K.

Next, genomic DNA was extracted from 110 hybrid progeny of Chinook and SaM hop varieties stocked by Sapporo Breweries Ltd. and analyzed by RFLP using combinations of the aforementioned DIG-labeled probes and restriction enzymes EcoT22I or XbaI, and a search was conducted for breeding markers that can detect polymorphisms correlated with cohumulone ratio, colupulone ratio, farnesene contents and linalool contents. The genomic DNA extraction and genomic Southern hybridization were conducted according to the method described in Molecular cloning (Maniatis et al., 1989, Cold Spring Harbor Laboratory Press).

As a result, polymorphism detected with the combination of the cDNA clone 3D03 (hereinafter "3D03") from lupulin gland and restriction enzyme XbaI (hereinafter, "XbaI/3D03 polymorphism") was shown to be correlated with cohumulone ratio, colupulone ratio, farnesene contents and linalool contents, suggesting that it can be utilized as a breeding marker.

In Example 2 it was examined whether 3D03 can be utilized as a breeding marker correlated with cohumulone ratio, colupulone ratio, farnesene contents and linalool contents in a wide range of hop lines other than the hybrid progeny of the Chinook and SaM varieties. The nucleotide sequence of 3D03 is listed as SEQ ID NO: 1.

(Example 2) Correlation between cohumulone ratio, colupulone ratio, farnesene contents or linalool contents and XbaI/3D03 polymorphism:

Nine hop lines stocked by Sapporo Breweries Ltd. (Daimanshu, 980690, M916001003, Hokusenzairai, 0004B, 971174, 980573, Monica, M843501246) were selected and crossbred to obtain hybrid progeny, the cohumulone ratio, colupulone ratio, farnesene contents and linalool contents of the hop cones were examined, and their correlation with XbaI/3D03 polymorphism was determined.

The cross breeding was carried out with the combinations Daimanshu × 980690, Daimanshu × M916001003, Hokusenzairai × 980690, 0004B × 971174, 0004B × 980573, Monica × M843501246, Monica × 980573, and the cohumulone ratio, colupulone ratio, farnesene contents and linalool contents in the cones of the obtained hybrid progeny were examined.

Correlation between the XbaI/3D03 polymorphism and cohumulone ratio, colupulone ratio, farnesene contents and linalool contents was judged by dividing the hybrid progeny obtained by cross breeding into groups based on XbaI/3D03 polymorphism, calculating the mean values for the component contents in each group, and determining statistically significant difference by T test. For the T test, the difference in variance of measured values among the XbaI/3D03 polymorphisms was investigated by F test for each hop line combination, and homoskedasticity was assumed when no difference in variance was found, while variance was assumed to be unequal when a difference in variance was found. The contribution ratio representing the effect of XbaI/3D03 polymorphisms on cohumulone ratio, colupulone ratio, farnesene contents and linalool contents was calculated as genetic variance/population variance. The genetic variance was the dispersion for a theoretical population experiencing no factors other than the subject genotype 3D03 (no influence by other gene loci or environment). Specifically, if the mean value for the parent population is represented as XP, the population variance as VP, the mean value for a population with genotypes 1, 2, ... as X1, X2, ... and the number of individuals in each genotype as N1, N2, then the genetic variance ($V_G$) and contribution ratio (H) may be calculated by the following formulas.

$$\text{Genetic variance } (V_G) = \left( (X_P - X_1)^2 \times N_1 + (X_P - X_2)^2 \times N_2 + \ldots \right) / (N_1 + N_2 + \ldots)$$

$$\text{Contribution ratio } (H) = V_G / V_P$$

Fig. 1 is an image showing XbaI/3D03 polymorphism, obtained by digestion of genomic DNA of different hop varieties with restriction enzyme XbaI, and genomic Southern hybridization. The hybrid progeny obtained by the different cross breeding combinations exhibited five different patterns, ee-type, ef-type, eg-type, fg-type and gg-type.

Table 1 shows the correlation between XbaI/3D03 polymorphism and cohumulone ratio.

[Table 1]

| | ee | | | ef | | | eg | | | fg | | | gg | | | Contribution ratio |
|---|---|---|---|---|---|---|---|---|---|---|---|---|---|---|---|---|
| | Average value (%) | Significant difference | Size of population | Average value (%) | Significant difference | Size of population | Average value (%) | Significant difference | Size of population | Average value (%) | Significant difference | Size of population | Average value (%) | Significant difference | Size of population | |
| Daimarishu × 980690 | 22.9 | a | 7 | 36.6 | ab | 2 | 24.4 | a | 12 | 34.7 | b | 9 | | | | 61.49 |
| Daimanshu × M916001003 | | | | | | | 22.7 | a | 17 | 33.6 | b | 8 | | | | 64.38 |
| Hokusenzairai × 980690 | 29.2 | ab | 6 | 36.3 | ab | 5 | 26.2 | a | 9 | 39.0 | b | 13 | | | | 49.10 |
| 0004B × 971174 | | | | | | | | | | 36.2 | a | 9 | 26.8 | b | 28 | 45.41 |
| 0004B × 980573 | | | | | | | | | | 41.3 | a | 18 | 26.8 | b | 18 | 67.57 |
| Monica × M843501246 | 58.5 | N.S. | 9 | | | | 29.8 | N.S. | 27 | | | | 28.3 | N.S. | 3 | 3.75 |
| Monica × 980573 | | | | 38.78 | b | 12 | 27.7 | a | 13 | 39.8 | b | 18 | 31.0 | ab | 7 | 38.22 |

The significant difference is shown at a significance level of 5% or less. The difference between a and b is significant; whereas, the differences between a and ab, and ab and b are not significant. N.S. represents no significant difference.

As a result, it was observed that the hybrid progeny represented by ef type and fg type tend to have high cohumulone ratio; whereas, the hybrid progeny represented by ee type, eg type and gg type tend to have low cohumulone ratio. These results suggested that a hop variety without an f-type genome has a lower cohumulone ratio than a hop variety containing at least one f-type genome.

Table 2 shows the correlation between XbaI/3D03 polymorphism and colupulone ratio.

[Table 2]

| | ee | | | ef | | | eg | | | fg | | | gg | | | Contribution ratio |
|---|---|---|---|---|---|---|---|---|---|---|---|---|---|---|---|---|
| | Average value (%) | Significant difference | Size of population | Average value (%) | Significant difference | Size of population | Average value (%) | Significant difference | Size of population | Average value (%) | Significant difference | Size of population | Average value (%) | Significant difference | Size of population | |
| Daimarishu × 980690 | 44.0 | a | 7 | 61.7 | ab | 2 | 46.2 | a | 12 | 59.5 | b | 9 | | | | 74.65 |
| Daimanshu × M916001003 | | | | | | | 46.5 | a | 17 | 57.6 | b | 8 | | | | 58.16 |
| Hokusenzairai × 980690 | 49.0 | ab | 6 | 62.0 | b | 5 | 47.1 | a | 9 | 63.4 | be | 13 | | | | 63.99 |
| 0004B × 971174 | | | | | | | | | | 58.5 | a | 9 | 48.5 | b | 28 | 48.46 |
| 0004B × 980573 | | | | | | | | | | 61.1 | a | 18 | 47.3 | b | 18 | 66.47 |
| Monica × M843501246 | 58.5 | N.S. | 9 | | | | 57.0 | N.S. | 27 | | | | 55.0 | N.S. | 3 | 3.69 |
| Monica × 980573 | | | | 65.8 | b | 12 | 55.3 | a | 13 | 65.4 | b | 18 | 56.2 | ab | 7 | 39.81 |

The significant difference is shown at a significance level of 5% or less. The difference between a and b is significant; whereas, the differences between a and ab, and ab and b are not significant. N.S. represents no significant difference.

As a result, it was observed that the hybrid progeny represented by ef type and fg type tend to have high colupulone ratio; whereas, the hybrid progeny represented by ee type, eg type and gg type tend to have low colupulone ratio. These results suggested that a hop variety without an f-type genome has a lower colupulone ratio than a hop variety containing at least one f-type genome.

Table 3 shows the correlation between XbaI/3D03 polymorphism and farnesene contents.

[Table 3]

| | ee | | | ef | | | eg | | | fg | | | gg | | | Contribution ratio |
|---|---|---|---|---|---|---|---|---|---|---|---|---|---|---|---|---|
| | Average value (%) | Significant difference | Size of population | Average value (%) | Significant difference | Size of population | Average value (%) | Significant difference | Size of population | Average value (%) | Significant difference | Size of population | Average value (%) | Significant difference | Size of population | |
| Daimanshu × 980690 | 0.02 | N.S. | 7 | 0.01 | N.S. | 2 | 0.02 | N.S. | 12 | 0.02 | N.S. | 9 | | | | 2.63 |
| Daimanshu × M916001003 | | | | | | | 0.49 | a | 17 | 0.24 | b | 8 | | | | 12.88 |
| Hokusenzairai × 980690 | 0.64 | N.S. | 6 | 0.30 | N.S. | 5 | 0.52 | N.S. | 9 | 0.35 | N.S. | 13 | | | | 15.92 |
| 0004B × 971174 | | | | | | | | | | 1.68 | a | 9 | 2.84 | b | 28 | 14.42 |
| 0004B × 980573 | | | | | | | | | | 2.50 | N.S. | 18 | 2.35 | N.S. | 18 | 0.24 |
| Monica × M843501246 | 2.84 | N.S. | 9 | | | | 3.56 | N.S. | 27 | | | | 3.38 | N.S. | 3 | 2.65 |
| Monica × 980573 | | | | 2.24 | N.S. | 12 | 4.32 | N.S. | 13 | 2.69 | N.S. | 18 | 3.15 | N.S. | 7 | 28.12 |

The significant difference is shown at a significance level of 5% or less. The difference between a and b is significant; whereas, the differences between a and ab, and ab and b are not significant. N.S. represents no significant difference.

As a result, it was observed that the hybrid progeny represented by ef type and fg type tend to have low farnesene contents; whereas, the hybrid progeny represented by ee type, eg type and gg type tend to have high farnesene contents. These results suggested that a hop variety without an f-type genome has a higher farnesene contents than a hop variety containing at least one f-type genome.

Table 4 shows the correlation between XbaI/3D03 polymorphism and linalool contents.

[Table 4]

| | ee | | | ef | | | eg | | | fg | | | gg | | | Contribution ratio |
|---|---|---|---|---|---|---|---|---|---|---|---|---|---|---|---|---|
| | Average value (%) | Significant difference | Size of population | Average value (%) | Significant difference | Size of population | Average value (%) | Significant difference | Size of population | Average value (%) | Significant difference | Size of population | Average value (%) | Significant difference | Size of population | |
| Daimanshu × 9880690 | 0.03 | N.S. | 7 | 0.08 | N.S. | 2 | 0.04 | N.S. | 12 | 0.04 | N.S. | 9 | | | | 15.17 |
| Daimanshu × M916001003 | | | | | | | 0.38 | a | 17 | 0.21 | b | 8 | | | | 11.68 |
| Hokusenzairai × 980690 | 0.46 | N.S. | 6 | 0.28 | N.S. | 5 | 0.36 | N.S. | 9 | 0.28 | N.S. | 13 | | | | 10.87 |
| 0004B × 971174 | | | | | | | | | | 1.05 | a | 9 | 1.58 | b | 28 | 11.92 |
| 0004B × 980573 | | | | | | | | | | 1.64 | N.S. | 18 | 1.90 | N.S. | 18 | 1.62 |
| Monica × M843501246 | 1.68 | N.S. | 9 | | | | 2.21 | N.S. | 27 | | | | 2.68 | N.S. | 3 | 7.59 |
| Monica × 980573 | | | | 1.35 | N.S. | 12 | 2.24 | N.S. | 13 | 1.41 | N.S. | 18 | 1.63 | N.S. | 7 | 27.48 |

The significant difference is shown at a significance level of 5% or less. The difference between a and b is significant; whereas, the differences between a and ab, and ab and b are not significant. N.S. represents no significant difference.

As a result, it was observed that the hybrid progeny represented by ef type and fg type tend to have low linalool contents; whereas, the cross progenies of ee type, eg type and gg type tend to have high linalool contents. These results suggested that a hop variety without an f-type genome has a higher linalool contents than a hop variety containing at least one f-type genome.

The results mentioned above suggest that whether cohumulone ratio, colupulone ratio, farnesene contents and linalool contents are high or low can be estimated by analyzing an arbitrarily chosen hop variety for an XbaI/3D03 polymorphism, and that a hop variety without an f-type genome is one having low cohumulone ratio and colupulone ratio, and high farnesene contents and linalool contents.

(Example 4) Cloning of genomic DNA fragment containing 3D03

Since it was suggested that a hop variety without f-type genome has low cohumulone ratio and colupulone ratio, and high farnesene contens and linalool contens, the nucleotide sequence of 3D03 was used for cloning of e-type, f-type and g-type genomic DNA fragments, and difference of nucleotide sequences thereof was analized. In this manner, identification of polymorphism at 3D03 gene locus was attempted.

First, the following primers were designed based on the sequences at both end regions of 3D03.

p3D03-F1 primer: 5'-ACTAGTTTCTCTCCACAATCTCTGC-3' (SEQ ID NO: 2)
p3D03-R1 primer: 5'-GGTGGAATATCATTATTGTGTCAGC-3' (SEQ ID NO: 3)

Next, genomic DNA was extracted from Chinook varieties carrying hetero genomes of e-type and f-type and a SaM variety carrying homologous genomes of g-type, PCR was conducted using the p3D03-F1 primer and p3D03-R1 primer, and the specifically amplified genomic DNA fragments were cloned. The nucleotide sequences were analyzed for the clones of the DNA fragments cloned from the Chinook variety genomic DNA and the clones of the DNA fragments cloned from the SaM variety genomic DNA. The PCR was conducted using PrimeSTAR HS DNA Polymerase by Takara Bio, Inc., according to the manufacturer's protocol. The nucleotide sequence analysis was carried out utilizing the Macrogen Data Analysis Service.

As a result, the DNA fragments cloned from the genomic DNA of breed Chinook were classified into two types of nucleotide sequences. One of them was cleaved by restriction enzyme HpaI, whereas the other one was not cleaved by restriction enzyme HpaI. Using the genomic DNA of breed Nugget having homologous genomes of e-type as a template, PCR was performed using p3D03-F1 primer and p3D03-R1 primer and whether the specifically amplified DNA fragments were cleaved by restriction enzyme HpaI or not was checked. As a result, it was found that the DNA fragment derived from the e-type genome is cleaved by restriction enzyme HpaI. Of the DNA fragments cloned from the genomic DNA of breed Chinook, the nucleotide sequences cleaved by restriction enzyme HpaI were identified as e-type and the nucleotide sequences not cleaved by restriction enzyme HpaI were identified as f-type. Furthermore, since breed SaM has homologous genomes of g type, the nucleotide sequences of the DNA fragments cloned form the genomic DNA of breed SaM were identified as g-type. The nucleotide sequence of e-type genomic DNA fragment (e-type g3DO3) is shown in SEQ ID NO: 4 of the sequence listing. The nucleotide sequence of f-type genomic DNA fragment (f-type g3DO3) is shown in SEQ ID NO: 5. The nucleotide sequence of g-type genomic DNA fragment (g-type g3DO3) is shown in SEQ ID No: 6.

Fig. 2 shows homology of the nucleotide sequences of e-type g3DO3, f-type g3DO3 and g-type g3DO3. According to the results, a single nucleotide polymorphism was found at the 623rd nucleotide of f-type g3DO3 indicated by the arrow in Fig. 2 (A), and at the 1820th nucleotide of f-type g3DO3 indicated by the arrow in Fig. 2 (B). It was demonstrated that a single nucleotide polymorphism at the 623rd nucleotide of f-type g3DO3 can be distinguished from e-type g3DO3 and g-type g3DO3 by cleavage with restriction enzyme PacI, and a single nucleotide polymorphism at the 1820th nucleotide of f-type g3DO3 can be distinguished from e-type g3DO3 and g-type g3DO3 by no cleavage with restriction enzyme HpaI.

(Example 5) Analysis of single nucleotide polymorphism found in f-type g3DO3

Since it had been determined that f-type g3DO3 contains a single nucleotide polymorphism that can be distinguished by no cleavage with restriction enzyme HpaI, it was then investigated whether this single nucleotide polymorphism can be utilized to screen for hop varieties with low cohumulone ratio and colupulone ratio, and high farnesene contents and linalool contents, in the same manner as the XbaI/3D03 polymorphism.

First, the genomic DNA of 9 hop lines (Daimanshu, 980690, M916001003, Hokusenzairai, 0004B, 971174, 980573, Monica, M843501246) were used for PCR-RFLP analysis using a combination of restriction enzyme HpaI and the following primer set consisting of p3DO3-F2 primer and p3DO3-R2 primer, and the resulting polymorphism (hereinafter referred to as "HpaI /g3D03 polymorphism") was detected.

p3D03-F2 primer: 5'-TGCTGTTTCTAAGGGATTCG-3' (SEQ ID NO: 7)
p3D03-R2 primer: 5'-TTTCCAGGACCTATTCTCTG-3' (SEQ ID NO: 8)

Specifically, DNA was synthesized by PCR using the genomic DNA of the 9 hop lines as template with p3D03-F2 primer and p3D03-R2 primer, and then the DNA was digested with restriction enzyme HpaI, the digested DNA fragments were fractionated by 1% agarose gel electrophoresis, and the band pattern of the fractionated DNA fragments in the agarose gel was examined. Detection of an approximately 1304 bp band in analysis of the HpaI/g3D03 polymorphism means that the genomic DNA has the f-type genome, while detection of an approximately 1138 bp band means that the genomic DNA has the 1-type genome.

Fig. 3 shows a typical band pattern for HpaI/g3D03 polymorphism, obtained by PCR-RFLP analysis of hop varieties, and Fig. 4 shows HpaI/g3D03 polymorphism obtained by analysis of the genomic DNA of the 9 hop lines.

The results suggested that Daimanshu, Hokusenzairai, 971174 and 980573 are hop lines with the f-type genome, 0004B, Monica, 980690, M916001003 and M843501246 are hop lines without the f-type genome.

Next, genomic DNA from the same 9 hop lines was used for RFLP analysis with a combination of restriction enzyme XbaI and 3D03, and the obtained XbaI/g3D03 polymorphisms were examined to confirm whether or not the determined results based on HpaI/g3D03 polymorphism were correct.

Specifically, the genomic DNA of each of the 9 hop lines was cleaved with restriction enzyme XbaI, the digested genomic DNA fragments were fractionated by 1% agarose gel electrophoresis, the fractionated genomic DNA fragments were transferred to a nylon membrane, labeled 3D03 was used as a probe for hybridization, and the hybridized bands were detected based on the probe labeling.

Fig. 5 is an image showing XbaI/g3D03 polymorphism, obtained by genomic analysis of the 9 hop lines.

The results suggested that Daimanshu, Hokusenzairai, 971174 and 980573 are hop lines with the f-type genome, 0004B, Monica, 980690, M916001003 and M843501246 are hop lines without the f-type genome.

The results described above demonstrated that analysis of HpaI/g3D03 polymorphism, like analysis of XbaI/g3D03 polymorphism, can determine whether or not a hop variety has the f-type genome, thus suggesting that it can be used as a screening method for hop varieties with low cohumulone ratio and colupulone ratio, and high farnesene contents and linalool contents.

**Industrial Applicability**

According to the invention it is possible to provide a breeding marker for hop varieties with low cohumulone ratio, the breeding marker also being usable as a breeding marker for hop varieties with low colupulone ratio, and for hop varieties with high farnesene contents and/or linalool contents. According to the invention it is also possible to objectively and rapidly screen for hop varieties with low cohumulone ratio and colupulone ratio, and high farnesene contents and linalool contents.

## Claims

1. A breeding marker suitable for screening of hop varieties with a low ratio of cohumulone contents relative to $\alpha$ acid contents in hops, represented by any one of the following (a) to (d):

   (a) a polynucleotide consisting of a nucleotide sequence of 20 to 3434 contiguous nucleotides including nucleotide No. 623 of the nucleotide sequence set forth in SEQ ID NO: 5 (wherein said nucleotide is adenine);
   (b) a polynucleotide consisting of the sequence complementary to the polynucleotide of (a);
   (c) a polynucleotide consisting of a nucleotide sequence of 20 to 3434 contiguous nucleotides including nucleotide No.1820 of the nucleotide sequence set forth in SEQ ID NO: 5 (wherein said nucleotide is adenine);
   (d) a polynucleotide consisting of the sequence complementary to the polynucleotide of (c) .

2. The breeding marker according to claim 1, which is suitable for screening of hop varieties with a low ratio of colupulone contents relative to $\beta$ acid contents in hops.

3. The breeding marker according to claim 1 or 2, which is suitable for screening of hop varieties with high contents of farnesene and/or linalool.

4. A screening method for hop varieties with a low ratio of cohumulone contents relative to $\alpha$ acid contents in hops which comprises the steps of (a) to (d):

   (a) extracting genomic DNA from a hop variety specimen;
   (b) digesting the genomic DNA with the restriction enzyme XbaI to obtain digested genomic DNA fragments;
   (c) separating the digested genomic DNA fragments and detecting genomic DNA fragments that hybridize to the breeding marker of any one of claims 1 to 3; and
   (d) judging that the variety has a low cohumulone ratio when a genomic DNA fragment of about 8.6 kbp is not detected in step (c).

5. A method for screening of hop varieties with a low ratio of cohumulone contents relative to $\alpha$ acid contents in hops which comprises the steps of (a) and (b):

   (a) extracting genomic DNA from a hop variety specimen; and
   (b) identifying a genomic DNA polymorphism at the $623^{rd}$ nucleotide position of the nucleotide sequence set forth in SEQ ID NO: 5.

6. The screening method according to claim 5, wherein step (b) comprises the steps of (i) to (iii):

   (i) a PCR step in which DNA is synthesized by PCR using the aforementioned genomic DNA as template, a primer consisting of a nucleotide sequence of 18 to 50 contiguous nucleotides located toward the 5'-end from the $623^{rd}$ nucleotide of the nucleotide sequence set forth in SEQ ID NO: 5 and a primer consisting of the sequence complementary to a nucleotide sequence of 18 to 50 contiguous nucleotides located toward the 3'-end from the $623^{rd}$ nucleotide of the nucleotide sequence set forth in SEQ ID NO: 5;
   (ii) digesting the DNA synthesized in step (i) with the restriction enzyme PacI; and
   (iii) judging that the variety has a low cohumulone ratio when the DNA is not cleaved with the restriction enzyme PacI in step (ii).

7. A method for screening of hop varieties with a low ratio of cohumulone contents relative to $\alpha$ acid contents in hops which comprises the steps of (a) and (b):

   (a) extracting genomic DNA from a hop variety specimen; and
   (b) identifying a genomic DNA polymorphism at the $1820^{th}$ nucleotide position of the nucleotide sequence set forth in SEQ ID NO: 5.

8. The screening method according to claim 7, wherein the step (b) comprises the steps of (i) to (iii):

   (i) a PCR step in which DNA is synthesized by PCR using the aforementioned genomic DNA as template, a primer consisting of a nucleotide sequence of 18 to 50 contiguous nucleotides located toward the 5'-end from the $1820^{th}$ nucleotide of the nucleotide sequence set forth in SEQ ID NO: 5 and a primer consisting of the sequence complementary to a nucleotide sequence of 18 to 50 contiguous nucleotides located toward the 3'-end from the 1820$^{th}$ nucleotide of the nucleotide sequence set forth in SEQ ID NO: 5;
(ii) digesting the DNA synthesized in step (i) with the restriction enzyme HpaI; and
(iii) judging that the variety has a low cohumulone ratio when the DNA is cleaved with the restriction enzyme HpaI in step (ii).

**9.** The screening method according to claim 7, wherein the step (b) comprises the steps of (i) to (iii):

(i) a PCR step in which DNA is synthesized by PCR using the aforementioned genomic DNA as template, a primer including the nucleotide sequence set forth in SEQ ID NO: 7 and a primer including the nucleotide sequence set forth in SEQ ID NO: 8;
(ii) digesting the DNA with the restriction enzyme HpaI, and
(iii) judging that the variety has a low cohumulone ratio when a DNA fragment of about 1304 bp is not contained in the DNA fragments obtained by step (ii).

**10.** The screening method according to any one of claims 4 to 9, which is a method for screening of hop varieties with a low ratio of colupulone contents relative to β acid contents in hops.

**11.** The screening method according to any one of claims 4 to 9, which is a method for screening of hop varieties with high contents of farnesene and/or linalool.

**12.** A primer set suitable for screening of hop varieties with a low ratio of cohumulone contents relative to α acid contents in hops, the primer set comprising

(i) a primer containing the nucleotide sequence set forth in SEQ ID NO: 7; and
(ii) a primer containing the nucleotide sequence set forth in SEQ ID NO: 8.

**Patentansprüche**

**1.** Züchtungsmarker, der zum Screenen von Hopfensorten mit einem niedrigen Verhältnis des Cohumulongehalts zum Gehalt an α-Säure in Hopfen geeignet ist, dargestellt durch eine der Folgenden von (a) bis (d):

(a) ein Polynucleotid bestehend aus einer Nucleotidsequenz von 20 bis 3434 zusammenhängenden Nucleotiden einschließlich des Nucleotids Nr. 623 der Nucleotidsequenz nach SEQ ID NO:5 (in der das Nucleotid ein Adenin ist);
(b) ein Polynucleotid bestehend aus der Sequenz, die komplementär zu dem Polynucleotid nach (a) ist;
(c) ein Polynucleotid bestehend aus einer Nucleotidsequenz von 20 bis 3434 zusammenhängenden Nucleotiden einschließlich des Nucleotids Nr. 1820 der Nucleotidsequenz nach SEQ ID NO:5 (in der das Nucleotid ein Adenin ist);
(d) ein Polynucleotid bestehend aus der Sequenz, die komplementär zum Polynucleotid nach (c) ist.

**2.** Züchtungsmarker nach Anspruch 1, der zum Screenen von Hopfensorten mit einem niedrigen Verhältnis des Colupulongehalts zum Gehalt an β-Säure im Hopfen geeignet ist.

**3.** Züchtungsmarker nach Anspruch 1 oder 2, der zum Screenen von Hopfensorten mit einem hohen Gehalt an Farnesen und/oder Linalool geeignet ist.

**4.** Screeningverfahren für Hopfensorten mit einem niedrigen Verhältnis des Cohumulongehalts zum α-Säuregehalt im Hopfen, das die Schritte (a) bis (d) umfasst:

(a) Extraktion der genomischen DNA aus der Probe der Hopfensorte;
(b) Verdau der genomischen DNA mit dem Restriktionsenzym XbaI, um verdaute genomische DNA-Fragmente zu erhalten;
(c) Trennung der verdauten genomischen DNA-Fragmente und Nachweis genomischer DNA-Fragmente, die mit dem Züchtungsmarker nach einem der Ansprüche 1 bis 3 hybridisieren; und
(d) Auswertung, dass die Sorte einen niedrigen Cohumulonanteil besitzt, wenn in Schritt (c) kein genomisches DNA-Fragment von etwa 8,6 kBp nachgewiesen wird.

**5.** Verfahren zum Screenen auf Hopfensorten mit einem niedrigen Verhältnis des Cohumulongehalts zum α-Säuregehalt im Hopfen, das die Schritte (a) und (b) umfasst:

(a) Extraktion der genomischen DNA aus einer Probe einer Hopfensorte; und
(b) Identifizierung eines Polymorphismus in der genomischen DNA an der 623sten Nucleotidposition der Nucleotidsequenz nach SEQ ID NO:5.

**6.** Screeningverfahren nach Anspruch 5, wobei Schritt (b) die Schritte (i) bis (iii) umfasst:

(i) einen PCR-Schritt, bei dem durch die PCR DNA synthetisiert wird unter Verwendung der vorgenannten genomischen DNA als Matrize, eines Primers bestehend aus einer Nucleotidsequenz von 18 bis 50 zusammenhängenden Nucleotiden, die sich in Richtung 5'-Ende ausgehend vom 623sten Nucleotid der Nucleotidsequenz nach SEQ ID NO:5 befinden, und eines Primers bestehend aus einer Sequenz, die komplementär zur Nucleotidsequenz von 18 bis 50 zusammenhängenden Nucleotiden ist, die sich in Richtung 3'-Ende ausgehend vom 623sten Nucleotid der Nucleotidsequenz nach SEQ ID NO:5 befinden;
(ii) Verdau der nach Schritt (i) synthetisierten DNA mit dem Restriktionsenzym PacI; und
(iii) Auswertung, dass die Sorte einen niedrigen Cohumulonanteil besitzt, wenn die DNA in Schritt (ii) nicht mit dem Restriktionsenzym PacI gespalten wird.

**7.** Verfahren zum Screenen auf Hopfensorten mit einem niedrigen Verhältnis des Cohumulongehalts zum α-Säuregehalt im Hopfen, das die Schritte (a) und (b) umfasst:

(a) Extraktion der genomischen DNA aus der Probe einer Hopfensorte; und
(b) Identifizierung eines Polymorphismus in der genomischen DNA an der 1820sten Nucleotidposition der Nucleotidsequenz nach SEQ ID NO:5.

**8.** Screeningverfahren nach Anspruch 7, wobei der Schritt (b) die Schritte von (i) bis (iii) umfasst:

(i) einen PCR-Schritt, bei dem durch die PCR DNA synthetisiert wird unter Verwendung der vorgenannten genomischen DNA als Matrize, eines Primers, bestehend aus einer Nucleotidsequenz von 18 bis 50 zusammenhängenden Nucleotiden, die sich in Richtung 5'-Ende ausgehend vom 1820sten Nucleotid der Nucleotidsequenz nach SEQ ID NO:5 befinden, und eines Primers bestehend aus der Sequenz, die komplementär zur Nucleotidsequenz von 18 bis 50 zusammenhängenden Nucleotiden ist, die sich in Richtung 3'-Ende ausgehend vom 1820sten Nucleotid der Nucleotidsequenz nach SEQ ID NO:5 befinden;
(ii) Verdau der nach Schritt (i) synthetisierten DNA mit dem Restriktionsenzym HpaI; und
(iii) Auswertung, dass die Sorte einen niedrigen Cohumulonanteil besitzt, wenn die DNA in Schritt (ii) mit dem Restriktionsenzym HpaI gespalten wird.

**9.** Screeningverfahren nach Anspruch 7, wobei Schritt (b) die Schritte von (i) bis (iii) umfasst:

(i) einen PCR-Schritt, bei dem durch die PCR DNA synthetisiert wird unter Verwendung der vorgenannten genomischen DNA als Matrize, eines Primers, der die Nucleotidsequenz nach SEQ ID NO:7 beinhaltet, und eines Primers, der die Nucleotidsequenz nach SEQ ID NO:8 beinhaltet;
(ii) Verdau der DNA mit dem Restriktionsenzym HpaI; und
(iii) Auswertung, dass die Sorte einen niedrigen Cohumulonanteil besitzt, wenn kein DNA-Fragment von etwa 1304 Bp in den durch Schritt (ii) erhaltenen DNA-Fragmenten gefunden wird.

**10.** Screeningverfahren nach einem der Ansprüche 4 bis 9, bei dem es sich um ein Screeningverfahren auf Hopfensorten mit einem niedrigen Verhältnis des Colupulongehalts zum β-Säuregehalt im Hopfen handelt.

**11.** Screeningverfahren nach einem der Ansprüche 4 bis 9, bei dem es sich um ein Screeningverfahren für Hopfensorten mit einem hohen Gehalt an Farnesen und/oder Limalool handelt.

**12.** Primersatz, der für ein Screeningverfahren auf Hopfensorten mit einem niedrigen Verhältnis des Cohumulongehalts zum α-Säuregehalt im Hopfen geeignet ist, wobei der Primersatz umfasst:

(i) einen Primer umfassend die Nucleotidsequenz nach SEQ ID NO:7; und
(ii) einen Primer umfassend die Nucleotidsequenz nach SEQ ID NO:8.

## Revendications

1. Marqueur de sélection permettant d'identifier par criblage des variétés de houblon ayant un bas rapport des teneurs de cohumulone par rapport aux teneurs d'acide $\alpha$ dans le houblon, représenté par l'un quelconque des polynucléotides (a) à (d) suivants :

   (a) un polynucléotide consistant en une séquence de nucléotides de 20 à 3434 nucléotides contigus incluant le nucléotide No. 623 de la séquence de nucléotides exposée dans SEQ ID NO : 5 (dans laquelle ledit nucléotide est l'adénine) ;
   (b) un polynucléotide consistant en la séquence complémentaire du polynucléotide de (a) ;
   (c) un polynucléotide consistant en une séquence de nucléotides de 20 à 3434 nucléotides contigus incluant le nucléotide No. 1820 de la séquence de nucléotides exposée dans SEQ ID NO : 5 (dans laquelle ledit nucléotide est l'adénine) ;
   (d) un polynucléotide consistant en la séquence complémentaire du polynucléotide de (c).

2. Marqueur de sélection selon la revendication 1, qui permet d'identifier par criblage des variétés de houblon ayant un bas rapport des teneurs de colupulone par rapport aux teneurs d'acide $\beta$ dans le houblon.

3. Marqueur de sélection selon les revendications 1 ou 2, qui permet d'identifier par criblage des variétés de houblon ayant des teneurs élevées de farnesène et/ou de linalol.

4. Procédé de criblage permettant d'identifier des variétés de houblon ayant un bas rapport des teneurs de cohumulone par rapport aux teneurs d'acide $\alpha$ dans le houblon qui comprend les étapes (a) à (d) :

   (a) extraire l'ADN génomique d'un spécimen d'une variété de houblon;
   (b) digérer l'ADN génomique avec une enzyme de restriction XbaI pour obtenir des fragments d'ADN génomique digérés ;
   (c) séparer les fragments d'ADN génomique digérés et détecter les fragments d'ADN génomique qui s'hybrident au marqueur de sélection selon l'une quelconque des revendications 1 à 3 ; et
   (d) juger que la variété a un bas rapport de cohumulone quand un fragment d'ADN génomique d'environ 8,6 kpb n'est pas détecté à l'étape (c).

5. Procédé de criblage permettant d'identifier des variétés de houblon ayant un bas rapport des teneurs de cohumulone par rapport aux teneurs d'acide $\alpha$ dans le houblon qui comprend les étapes (a) et (b) :

   (a) extraire l'ADN génomique d'un spécimen d'une variété de houblon ; et
   (b) identifier un polymorphisme dans l'ADN génomique à la $623^{ème}$ position nucléotidique de la séquence de nucléotides exposée dans SEQ ID NO : 5.

6. Procédé de criblage selon la revendication 5, dans lequel l'étape (b) comprend les étapes (i) à (iii):

   (i) une étape de PCR dans laquelle l'ADN est synthétisé par PCR en utilisant l'ADN génomique susmentionné en tant que matrice, une amorce consistant en une séquence de nucléotides de 18 à 50 nucléotides contigus s'étendant en direction de l'extrémité 5' à partir du $623^{ème}$ nucléotide de la séquence de nucléotides exposée dans SEQ ID NO : 5 et une amorce consistant en la séquence complémentaire d'une séquence de nucléotides de 18 à 50 nucléotides contigus s'étendant en direction de l'extrémité 3' à partir du $623^{ème}$ nucléotide de la séquence de nucléotides exposée dans SEQ ID NO : 5 ;
   (ii) digérer l'ADN synthétisé à l'étape (i) avec l'enzyme de restriction PacI; et
   (iii) juger que la variété a un bas rapport de cohumulone quand l'ADN n'est pas clivé avec l'enzyme de restriction PacI à l'étape (ii).

7. Procédé de criblage permettant d'identifier des variétés de houblon ayant un bas rapport des teneurs de cohumulone par rapport aux teneurs d'acide $\alpha$ dans le houblon qui comprend les étapes (a) et (b) :

   (a) extraire l'ADN génomique d'un spécimen d'une variété de houblon ; et
   (b) identifier un polymorphisme dans l'ADN génomique à la $1820^{ème}$ position nucléotidique de la séquence de nucléotides exposée dans SEQ ID NO : 5.

**8.** Procédé de criblage selon la revendication 7, dans lequel l'étape (b) comprend les étapes (i) à (iii):

(i) une étape de PCR dans laquelle l'ADN est synthétisé par PCR en utilisant l'ADN génomique susmentionné en tant que matrice, une amorce consistant en une séquence de nucléotides de 18 à 50 nucléotides contigus s'étendant en direction de l'extrémité 5' à partir du 1820$^{ème}$ nucléotide de la séquence de nucléotides exposée dans SEQ ID NO : 5 et une amorce consistant en la séquence complémentaire d'une séquence de nucléotides de 18 à 50 nucléotides contigus s'étendant en direction de l'extrémité 3' à partir du 1820$^{ème}$ nucléotide de la séquence de nucléotides exposée dans SEQ ID NO : 5 ;
(ii) digérer l'ADN synthétisé à l'étape (i) avec l'enzyme de restriction HpaI; et
(iii) juger que la variété a un bas rapport de cohumulone quand l'ADN est clivé avec l'enzyme de restriction HpaI à l'étape (ii).

**9.** Procédé de criblage selon la revendication 7, dans lequel l'étape (b) comprend les étapes (i) à (iii):

(i) une étape de PCR dans laquelle l'ADN est synthétisé par PCR en utilisant l'ADN génomique susmentionné en tant que matrice, une amorce comprenant la séquence de nucléotides exposée dans SEQ ID NO : 7 et une amorce comprenant la séquence de nucléotides exposée dans SEQ ID NO : 8 ;
(ii) digérer l'ADN avec l'enzyme de restriction HpaI; et
(iii) juger que la variété a un bas rapport de cohumulone quand un fragment d'ADN d'environ 1304 pb n'est pas contenu dans les fragments d'ADN obtenus à l'étape (ii).

**10.** Procédé de criblage selon l'une quelconque des revendications 4 à 9, qui est un procédé de criblage permettant d'identifier des variétés de houblon ayant un bas rapport des teneurs de colupulone par rapport aux teneurs d'acide $\beta$ dans le houblon.

**11.** Procédé de criblage selon l'une quelconque des revendications 4 à 9, qui est un procédé de criblage permettant d'identifier des variétés de houblon ayant une teneur élevée de farnesène et/ou de linalol.

**12.** Jeu d'amorces pouvant être utilisé pour identifier par criblage des variétés de houblon ayant un bas rapport des teneurs de cohumulone par rapport aux teneurs d'acide $\alpha$ dans le houblon, le jeu d'amorces comprenant

(i) une amorce contenant la séquence de nucléotides exposée dans SEQ ID NO : 7 ; et
(ii) une amorce contenant la séquence de nucléotides exposée dans SEQ ID NO : 8.

*Fig.1*

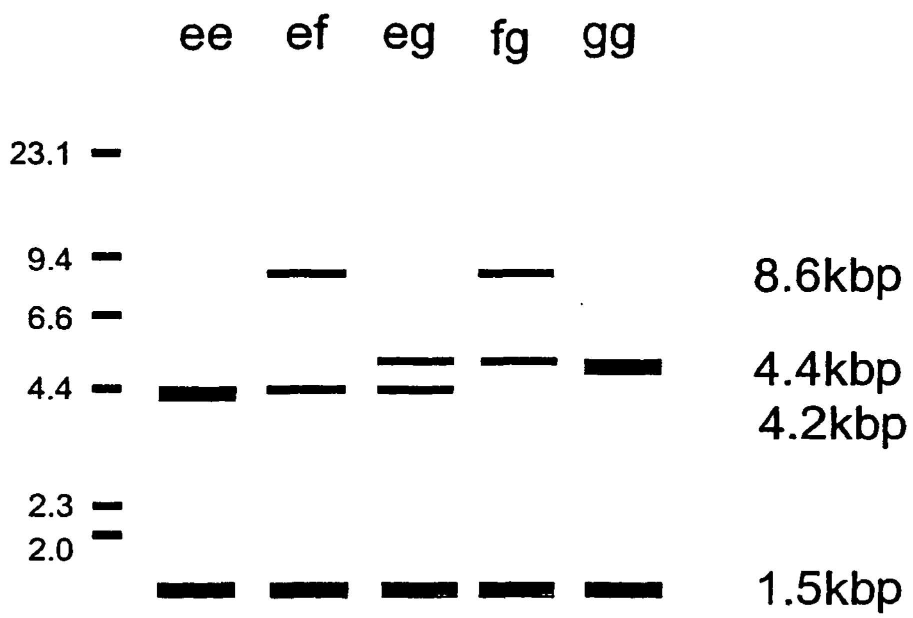

# Fig.2

(A)

```
                             PacI
gLytB-typeE.gnu  601: tttaatcttcattttattttttgattaatgttagtgttttcttcttcgtaatggctcaatttatatatttattaatattattatgattatttggatagAGGCTAGAGGAAATGAAAGTAG 720
gLytB-typeF.gnu  601: tttaatcttcattttattttttaattaatgttagtgttttcttcttcgtaatggctcaatttatatatttattaatattattatgattatttggatagAGGCTAGAGGAAATGAAAGTAG 720
gLytB-typeG.gnu  601: tttaatcttcattttattttttgattaatgttagtgttttcttcttcgtaatggctcaatttatatatttattaatattattatgattatttggatagAGGCTAGAGGAAATGAAAGTAG 720
                      ********************** *************************************************************************************************
                                            ↑
```

(B)

```
                                                                                  HpaI
gLytB-typeE.gnu 1795: gtaccataaaactcaaacataaatatcttataaattatcaaaattttttaaataaagttaacttttgtttctagaactagaatcctagattccactatcctagaataaacttctgatcag 1914
gLytB-typeF.gnu 1762: gtaccataaaactcaaacataaatatcttataaattatctaaattttttaaataaagt-aacttttgtttctagaactagaatcctagattccactatcctagaataaacttctgatcag 1880
gLytB-typeG.gnu 1795: gtaccataaaactcaaacataaatatcttataaattatcaaaattttttaaataaagttaacttttgtttctagaactacaatcctagattccactatcctagaataaacttctgatcag 1914
                      *****************************************.**************** ********************.****************************************
                                                                                    ↑
```

*Fig.3*

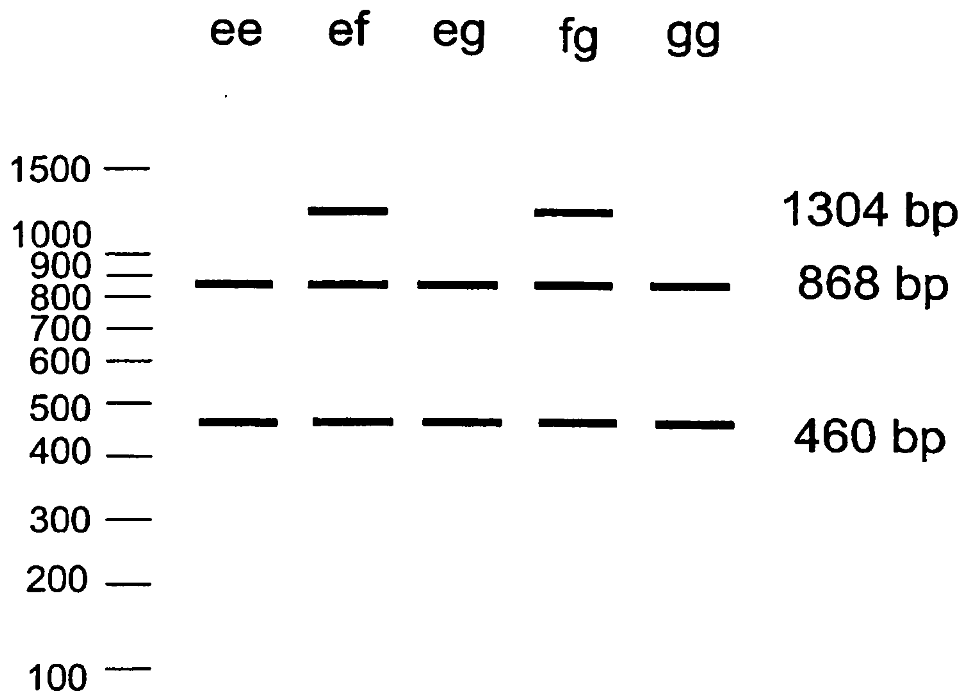
ee
ef
eg
fg
gg
1500
1000
900
800
700
600
500
400
300
200
100
1304 bp
868 bp
460 bp

Fig.4

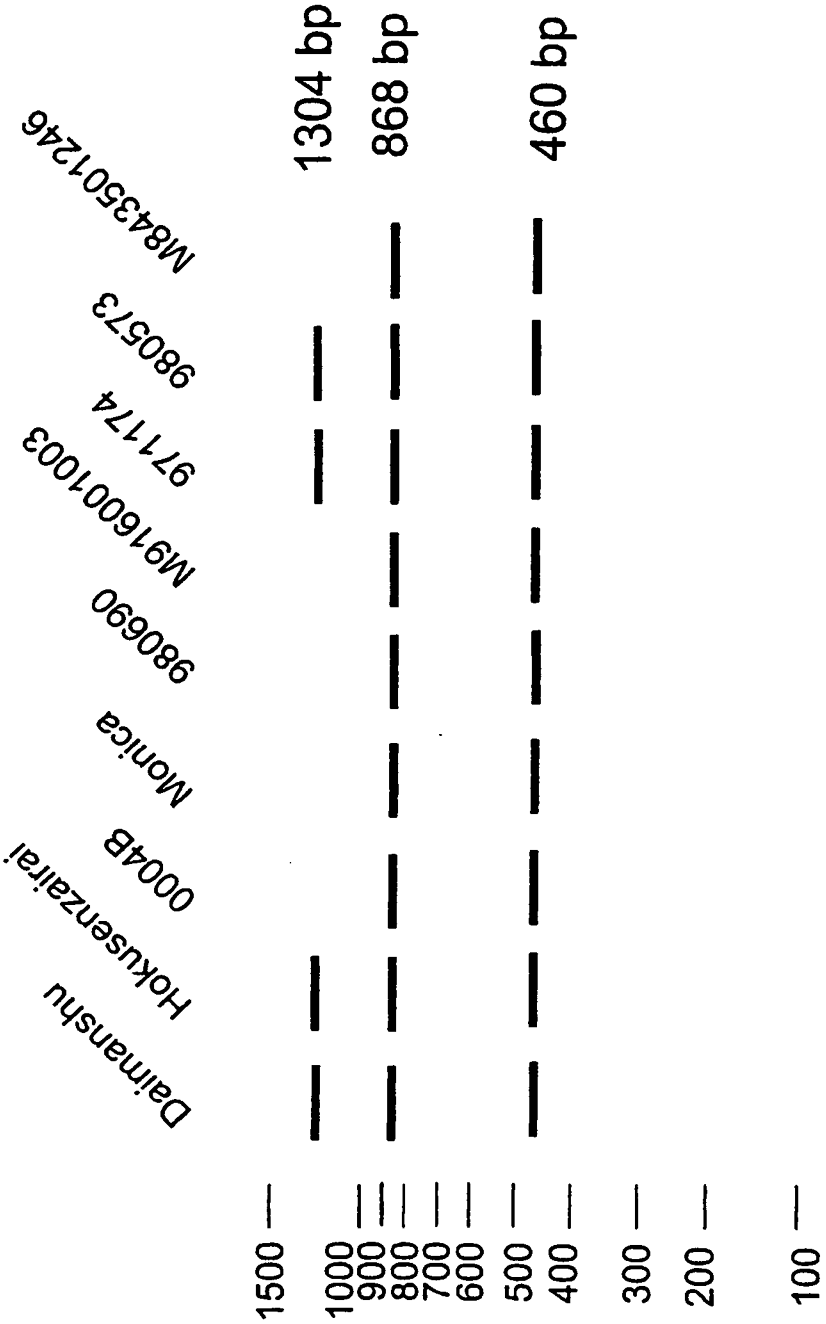
Daimanshu
Hokusenzairai
0004B
Monica
980690
M91600100З
971174
980573
M84350124б
1304 bp
868 bp
460 bp
1500
1000
900
800
700
600
500
400
300
200
100

Fig.5

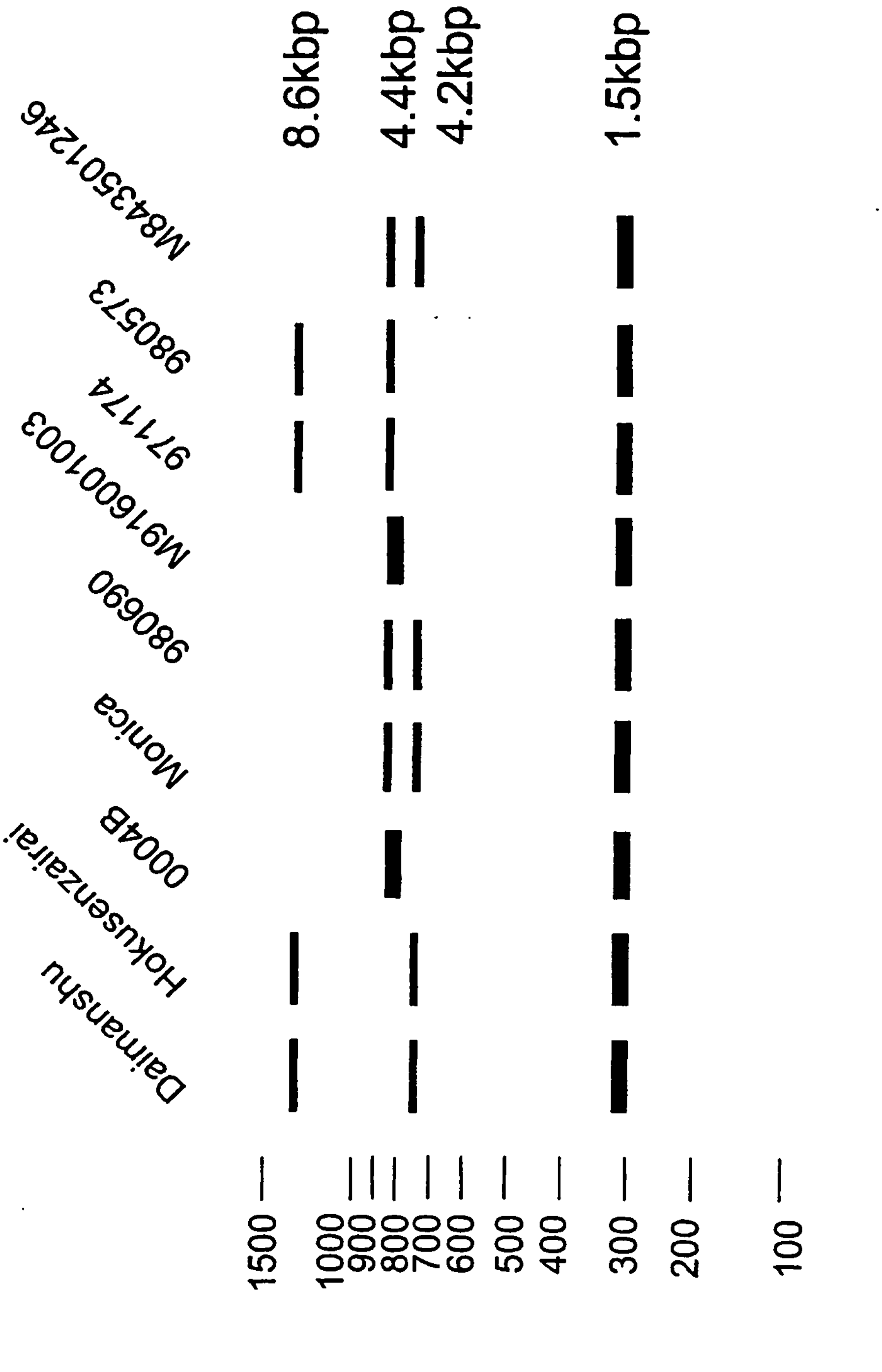
Daimanshu
Hokusenzairai
0004B
Monica
980690
M91600l003
971174
980573
M84350l246
8.6kbp
4.4kbp
4.2kbp
1.5kbp
1500
1000
900
800
700
600
500
400
300
200
100

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 07830755 A
- JP 2007071021 W
- JP 2006294798 A


### Non-patent literature cited in the description


- **Gerhaeuser.** *European Journal of Cancer,* 2005, vol. 41 (13), 1941-195.4
- **Peacock et al.** *European Brewing Convention monograph XXII Symposium on hops Zoeterwoude,* 1994, 247-250
- **Kowaka et al.** *EBC proceedings,* 1983, vol. 19, 71-78
- **Peacock et al.** *J. Agric. Food Chem.,* 1980, vol. 28, 774-777
- **Maniatis et al.** Molecular cloning. Cold Spring Harbor Laboratory Press, 1989